Europäisches Patentamt

**European Patent Office**

**Office européen des brevets**

(11) Numéro de publication:  **0 285 502 B1**

(12) **FASCICULE DE BREVET EUROPEEN**

(45) Date de publication de fascicule du brevet:  **27.04.94**

(21) Numéro de dépôt: **88400699.0**

(22) Date de dépôt: **23.03.88**

(51) Int. Cl.⁵: **C08K  3/08**, C08K 9/10, B22F 9/30

Le dossier contient des informations techniques présentées postérieurement au dépôt de la demande et ne figurant pas dans le présent fascicule.

(54) **Particules composites polymère/métal compactes, dispersions aqueuses desdites particules, leur procédé de préparation et leur application en biologie.**

(30) Priorité: **03.04.87 FR 8704684**

(43) Date de publication de la demande:
**05.10.88 Bulletin  88/40**

(45) Mention de la délivrance du brevet:
**27.04.94 Bulletin  94/17**

(84) Etats contractants désignés:
**AT BE CH DE ES FR GB GR IT LI LU NL SE**

(56) Documents cités:
**EP-A- 0 125 617
US-A- 3 167 525
US-A- 4 123 396**

(73) Titulaire: **SOCIETE PROLABO
12, rue Pelée
F-75011 Paris(FR)**

(72) Inventeur: **Charmot, Dominique
18/20, rue Mathis
F-75019 - Paris(FR)**

(74) Mandataire: **Ahner, Francis et al
CABINET REGIMBEAU
26, avenue Kléber
F-75116 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

**Description**

La présente invention a pour objet des particules composites compactes à base d'une matrice de polymère renfermant des inclusions de métal, lesdites particules composites étant de préférence calibrées et se présentant telles quelles ou en dispersion notamment dans l'eau, leur procédé de préparation et leur application en biologie.

Des particules de polymère imprégnées de métal à l'état de valence O sont décrites dans les brevets américains n° 4197220 et n° 4123396. Il s'agit de particules de polyvinylpyridine hydrophiles et microporeuses que l'on imprègne de métal par complexation d'ions métalliques suivie d'une réduction au borohydrure. La microporosité du support ainsi que l'état de division du métal précipité à l'intérieur des pores en font un matériau intéressant pour l'hydrogénation catalytique des oléfines. D'autres applications sont également citées comme l'imagerie médicale (particules opaques aux électrons en microscopie électronique) ou les techniques de séparation de protéines.

L'inconvénient présenté par de telles particules consistent en ce qu'elles sont poreuses et contiennent en surface du métal.

En effet dans des applications qui font intervenir des substances biologiques (protéines, etc...), des cellules ou des médicaments, il est très important que le métal soit totalement encapsulé dans le polymère de la particule et absent de la surface.

Ainsi en génie enzymatique, il est connu que certaines enzymes sont inhibées au contact d'ions métalliques provenant du support. Par exemple, on montre que la chymotrypsine immobilisée sur de la poudre de nickel perd rapidement son activité (MUNRO.P.A et al, Biotechnology and Bioengeneering. Vol XIX, 101-124, (1977)).

Un autre effet indésirable concerne les phénomènes d'adsorption irréversible d'hormones (T3, T4) ou de stéroides sur des supports magnétiques à base d'oxyde de fer, ce qui gène considérablement les dosages de type radioimmunoassays (RIA).

Enfin, en culture cellulaire, la présence d'ions métalliques issus du support peuvent également perturber l'adhésion des cellules.

La préparation de colloides metalliques en milieu organique est connue depuis les travaux de HESS et PARKER (J.Appl. Poly. Sci, Vol 10, 1915-1927, (1966)) et J.R. THOMAS (Brevet américain n° 32228881) ainsi que T.W.SMITH (Brevet américain n° 4252671 à 4252678). La technique consiste à décomposer thermiquement un dérivé métal carbonyle dans une solution organique de polymère. Le ferrofluide obtenu est une dispersion colloidale de particules métalliques de $10^{-6}$ à $10^{-5}$ mm dans un solvant organique. Le polymère est adsorbé à la surface des grains métalliques et assure la stabilité colloidale de la suspension. Lorsque ce ferrofluide est exposé à l'atmosphère ambiante, les gruins métalliques, bien qu'étant confinés dans une phase organique hydrophobe, s'oxydent rapidement et perdent leurs propriétés magnétiques (GRIFFITHS et al., J.Appl Phy., Vol 50 (11), Nov. 1979, p. 7108).D'autre part, à cause de leur taille généralement inférieure à 3 $10^{-5}$ mm, ces grains métalliques surfactés par le polymère ne peuvent être séparés du solvant organique (par aimantation, dans le cas de ferrofluide de fer ou de cobalt) sans détruire la stabilité colloidale. Enfin, ces grains de métal surfactés sont totalement incompatibles avec les milieux aqueux et ne peuvent pas en conséquence être utilisés dans les applications décrites précédemment.

La Demanderesse a decrit dans son brevet européen n° 38.730 des latex de particules de polymère vinylaromatique hydrophobe renfermant une charge magnétisable ; ceux-ci sont obtenus par dispersion de la charge magnétique dans une phase organique formée d'un amorceur organosoluble, du monomère vinylaromatique et/ou d'un composé organique insoluble dans l'eau, puis mélange de la dispersion avec une solution aqueuse d'émulsifiant, homogénéisation et enfin polymérisation ; un tel procédé présente l'inconvénient de ne pouvoir être utilisé que pour la préparation de latex de particules non calibrées, c'est-à-dire présentant une distribution granulométrique étalée.

Il est bien connu que lorsqu'ils sont utilisés comme phase solide en biologie, les latex doivent être de préférence calibrés.

La Demanderesse a trouvé un procédé permettant de préparer des particules compactes composites polymère/métal calibrées ou non dans lesquelles le métal est totalement encapsulé (inclus) dans le polymère, conserve son état de valence O ainsi que ses propriétés magnétiques (s'il s'agit d'un métal magnétisable tel que le fer ou le cobalt) au cours du temps.

Le métal n'est plus accessible à la surface des particules et n'est plus en mesure d'interagir avec des substances ancrées à la surface du polymère.

La présente invention a pour objet un procédé de préparation de particules compactes composites polymère/métal comprenant les étapes suivantes :

2

1) on disperse dans un liquide organique, solvant de complexes métalcarbonyles, organocarbonyles ou hydrocarbonés décomposables thermiquement à une température inférieure au point d'ébullition dudit liquide organique, des particules anhydres de polymère portant des sites nucléophiles coordinables auxdits complexes, la taille desdites particules étant de l'ordre de 0,1 micron à 1 mm, de préférence de l'ordre de 0,5 à 100 microns, la nature dudit polymère constituant lesdites particules étant telle que lesdites particules sont susceptibles de gonfler de 0,1 à 50 fois, de préférence de 3 à 10 fois, leur volume en présence dudit liquide organique ;

2) on introduit dans la dispersion de particules de polymère gonflées ainsi obtenue, un complexe métal carbonyle, organocurbonyle ou hydrocarboné soluble dans ledit liquide organique, selon un rapport pondéral métal dudit complexe/polymère de l'ordre de 0,5/100 à 200/100, de préférence de l'ordre de 3/100 à 35/100, et on effectue une opération de thermolyse dudit complexe, cette opération ayant lieu sous atmosphère réductrice lorsque ledit complexe est un complexe métal hydrocarboné

3) on élimine ledit liquide organique gonflant

4) éventuellement on redisperse lesdites particules composites compactes ainsi obtenues dans un liquide non gonflant dudit polymère.

Le polymère pouvant être mis en oeuvre peut être tout polymère réticulé portant des sites nucléophiles susceptibles de se coordiner aux complexes carbonyles, organocarbonyles ou hydrocarbonés dudit métal, ledit polymère devant en outre être susceptible de gonfler en présence d'un liquide organique solvant d'un desdits complexes, jusqu'à un volume tel qu'il devient accessible aux molécules dudit complexe.

Parmi ces polymères réticulés on peut citer les copolymères dérivés de ;

- 30 à 99 %, de préférence 50 à 95 %, en poids d'au moins un monomère monoethylenique.

On peut citer parmi les monomères monoethyléniques :

. le styrène et ses dérivés (vinyltoluene, ethylvinylbenzene...)

. les esters et amides de l'acide (meth) acrylique tels que le methacrylate de methyle, l'acrylate de butyle, (meth) acrylamide,

. l'acide (meth) acrylique et les diacides monoethyleniques tels que l'acide maleique, l'acide itaconique

. la vinylpyrrolidone

. les esters vinyliques (acétate de vinyle, propionate de vinyle

. les chlorures de vinyle et vinylidiène.

- 0,5 à 50 % de préférence 2 à 20 %, en poids d'au moins un monomère polyéthylénique réticulable.

On peut citer parmi les monomères polyethyléniques :

. le divinylbenzène et ses dérivés

. les dienes conjugués (butadiene,...)

. les dérivés polyallyliques (tetraallylethylene, ...)

. les (meth) acrylates de polyols (dimethacrylate d'éthylene glycol ...)

. le methylene-bis(acrylamide)

. l'acide-bis(acrylamido)acétique

- et 0,5 à 30 %, de preférence 3 à 30 %, en poids d'au moins un monomère nucléophile coordinable.

On peut citer parmi les monomères nucléophiles coordinables :

. les vinylpyridines (2-vinylpyridine, 4-vinylpyridine, 2-methyl, 5-vinylpyridine, 2-5 divinylpyridine,...)

. les di(ethyl)amino-alkyl(meth)acrylates

. les di(ethyl)amino-alkyl(meth)acrylamides,...

. l'allylamine

. l'éthylene imine

. le (meth)acrylonitrile

. le 1-vinylimidazole

. les dialkylaminométhyl styrènes

Lesdits copolymères peuvent être obtenus selon les techniques classiques de polymérisation en émulsion, microsuspension, suspension ou dispersion par voie radicaire.

Les particules de polymère mises en oeuvre à la première étape peuvent présenter une répartition granulométrique polydispersée ou monodispersée, c'est-à-dire être calibrées ou non calibrées. Le terme "calibrées" signifie de taille uniforme avec un écart type en diamètre inférieur à 5 %.

La nature et le faible taux de réticulation dudit polymère permet aux particules mises en oeuvre de gonfler d'une manière sensible tout en restant insolubles dans ledit liquide organique.

Le liquide organique est choisi de telle sorte qu'il gonfle les particules de polymère, ne décompose pas spontanément les complexes métal carbonyles, organocarbonyles ou hydrocarbures, et est inerte vis-à-vis de ceux-ci (il est préférable que ledit liquide organique ne soit pas electron-donneur).

Pour une meilleure réalisation industrielle, il est préférable que le point d'ébullition dudit liquide organique soit à pression atmosphérique supérieur à 80°C et de préférence supérieur à 100°C, ce afin d'éviter l'utilisation d'installations sous pression.

Parmi les liquides organiques pouvant favorablement être mis en oeuvre, on peut citer les dérivés aromatiques (toluene, ethylbenzene, xylenes, ...), les dérivés aromatiques chlorés (mono, di, et trichlorobenzene, ...), les hydrocarbures aliphatiques et cycliques (heptane, décane, cyclohexane, décaline, ...), les dialkyl ethers, les alcools (pentanol, cyclohexanol, ...), les esters (propionate de methyle, ...), etc...

La dispersion desdites particules de polymère dans le liquide organique s'effectue sans difficultés majeures ; la présence d'un agent émulsifiant n'est pas nécessaire car le gonflement important du polymère favorise les mécanismes de stabilisation par répulsion stérique.

La concentration en poids de particules de polymère dans le liquide organique peut varier entre 1 et 35 % suivant la capacité de gonflement du polymère. Une concentration de l'ordre de 1 à 20 % en poids est souhaitable, ce afin de conserver une viscosité modérée du milieu pour faciliter par exemple le dégagement ultérieur de monoxyde de carbone lorsque le complexe mis en oeuvre est un métal carbonyle.

A la deuxième étape, parmi les complexes métal carbonyles, organocarbonyles ou hydrocarbonés pouvant être mis en oeuvre, on peut citer les complexes carbonyles, organocarbonyles de type aliphatique, cycloaliphatique ou aromatique, hydrocarbonés de type aliphatique, cycloaliphatique ... des métaux des groupes VIa, VIIa et VIII de la Classification périodique des éléments, notamment ceux des métaux suivants chrome, molybdène, tungstène, manganèse, rhénium, fer, cobalt, rhodium, iridium, nickel, osmium, ruthénium.

A titre d'exemple on peut citer :
- les complexes métalcarbonyles suivants :
$Cr(CO)_6$ ; $Mo(CO)_6$ ; $W(CO)_6$
$(Mn)_2 (CO)_{10}$ ; $(Re)_2 (CO)_{10}$
$Fe(CO)_5$ ; $(Fe)_2 (CO)_9$ ; $(Fe)_3 (CO)_{12}$ ; $(CO)_2 (CO)_8$ ; $(Co)_4 (CO)_{12}$ ; $Ni(CO)_4$ ; $(Rh)_6 (CO)_{12}$ ; $(Rh)_4 (CO)_{12}$ ; $(Ir)_4 (CO)_{12}$ $(Os)_3 (CO)_{12}$ ; $(Ru)_3 (CO)_{12}$
- les complexes organocarbonyles suivants :
$(methylcyclopentadienyl)Mn(CO)_3$ ;
$(cyclopentadienyl)Mn(CO)_3$ ; $(cyclopentadienyl) Co(CO)_2$ ; $(cyclopentadienyl)Re(CO)_3$
- les complexes hydrocarbons suivants :
$(di- {}^5 -cyclopentadienyl)Ni$ ou $Fe$

A cette deuxième étape, l'introduction du complexe métallique est de préférence réalisée à température ambiante ou tout au moins à une température inférieure à celle de décomposition dudit complexe, ce pour éviter un brusque dégagement de monoxyde de carbone par exemple dans le cas des complexes carbonyles.

Le complexe est de préférence mis en oeuvre en totalité dès le début de la 2ème étape ; toutefois, une variante consiste à introduire de 15 % à 50 % dudit complexe au départ, puis à additionner le reste dudit complexe en continu sur une durée de 0,5 à 3 heures.

L'opération de thermolyse est réalisée à une température supérieure à la température de décomposition dudit complexe et de préférence voisine de celle du point d'ébullition dudit liquide organique, ce afin de favoriser l'élimination par exemple du monoxyde de carbone ; le déroulement de cette opération peut être suivi en mesurant par exemple le volume de monoxyde de carbone dégagé.

Ladite opération de thermolyse peut être réalisée sous atmosphère réductrice pour éviter une éventuelle oxydation du metal cette opération peut par exemple être conduite sous bullage d'hydrogène au sein du milieu réactionnel.

Lorsque le complexe mis en oeuvre est un complexe métal hydrocarboné par exemple le $(di- {}^5 -cyclopentadienyl)Ni$ ou $Fe$ (appelés nickelocène et ferrocène), cette opération est impérativement conduite sous atmosphère réductrice apportée par exemple par bullage d'hydrogène au sein du milieu réactionnel ; elle est de préférence conduite sous une pression d'hydrogène de 5 à 10 bar par exemple.

Lorsque le liquide organique ne gonfle les particules de polymère que lorsqu'il est porté à une certaine température appelée température théta (c'est le cas notamment du cyclohexane ou du décahydronaphtalène vis-à-vis des copolymères riches en motifs styreniques), il est bien entendu que la température de réalisation de la 2ème étape doit être supérieure à cette température théta.

Compte tenu de ces considérations, le domaine de température préférentiel de la réaction de thermolyse se situe généralement entre 80°C et 200°C.

Le temps nécessaire pour la décomposition totale du complexe est bien entendu fonction de la température mise en oeuvre et de la stabilité thermique dudit complexe ; généralement cette opération dure au moins 1 heure et n'excède par 24 heures.

4

EP 0 285 502 B1

Au cours de cette étape le complexe ajouté à la dispersion de particules de polymère gonflées se dissout d'abord à l'état moléculaire à l'extérieur et à l'intérieur des particules gonflées de liquide organique ; lorsque l'ensemble est porté à une température supérieure à celle de décomposition du complexe (correspondant de préférence à la température de reflux dudit liquide organique), le complexe se décompose ; la précipitation du métal à l'état de valence zéro est amorcée à l'intérieur des particules grâce aux sites nucléophiles du polymère ; les nucléi ainsi formés catalysent à leur tour la décomposition du complexe ; aucune formation de métal n'est observée dans la phase liquide organique en dehors des particules.

La teneur en métal des particules obtenues à la fin de cette deuxième étape, la grosseur des cristallites et leur répartition peuvent être contrôlées en ajustant les conditions opératoires.

La décomposition thermique des complexes s'amorçant au niveau des sites nucléophiles, la densité des nucléi métalliques est directement reliée au nombre de sites nucléophiles. Ainsi pour une même quantité de métal introduite, les cristallites de métal sont d'autant plus petites que la concentration en sites est plus élevée.

Les conditions opératoires décrites ci-dessus permettent d'aboutir à une taille de cristallites de l'ordre de $10^{-6}$ à $10^{-4}$ mm, et le plus souvent de l'ordre de $3\ 10^{-6}$ à $3\ 10^{-5}$ mm; Le taux de métal ainsi incorporé peut aller jusqu'à 67 % en poids, de préférence jusqu'à 26 % en poids, par rapport aux particules.

La troisième étape est celle d'élimination du liquide organique gonflant ; elle a pour but d'encapsuler le métal par le polymère, par passage des particules de polymère de l'étut gonflé à l'état compact la taille des particules composites obtenues correspondant quasiment celle des particules du polymère de depart.

Ledit liquide organique gonflant peut être éliminé du milieu réactionnel par les moyens classiques de séparation liquide-solide tels que la centrifugation, la filtration ou l'aimantation (lorsque le métal contenu dans les particules est magnétisable).

Le liquide organique gonflant restant dans ledites particules peut ensuite être éliminé par séchage.

Un autre mode d'élimination du liquide organique gonflant consiste, après séparation des particules du milieu réaction, à laver lesdites particules à l'aide d'un liquide non-gonflant du polymère et miscible au liquide organique gonflant ; cette opération de lavage peut être répétée plusieurs fois.

Le choix du liquide non gonflant est lié bien entendu à la nature du polymère. Ainsi lorsque ledit polymère est riche en motifs styréniques, les liquides non gonflants les plus usuels sont les monoalcools tels que le méthanol, l'éthanol, l'isopropanol et les diols tels que le monoethylène ou propylène glycol.

Si désiré, les particules composites en dispersion ainsi obtenues, peuvent être isolées par tout moyen connu tel que la centrifugation, la filtration ou l'aimantation (lorsque le métal contenu dans les particules composites est magnétisable).

Un mode de réalisation tout particulièrement intéressant du procédé de l'invention, consiste en la préparation de latex (dispersions aqueuses) de particules composites.

Ledit mode particulier consiste à réaliser l'élimination du liquide organique gonflant (3ème étape) par séparation des particules obtenues à la 2ème étape par centrifugation, filtration ou aimantation (lorsque le métal contenu est magnétisable), puis lavage des particules séparées, à l'aide d'un liquide non gonflant intermédiaire miscible avec le liquide organique gonflant et avec l'eau (cette opération de lavage pouvant être répétée plusieurs fois), à effectuer une nouvelle opération de séparation des particules composites ainsi obtenues et une nouvelle opération de lavage par de l'eau desdites particules composites ainsi séparées (cette opération de lavage pouvant être répétée plusieurs fois).

Le liquide non gonflant intermédiaire doit être miscible avec le liquide gonflant et avec l'eau, et ne pas être réactif avec le métal contenu dans les particules.

Ainsi lorsque le liquide organique gonflant est un dérivé aromatique (toluène, xylène) le milieu non gonflant intermédiaire peut être choisi parmi le monoalcools tels que méthanol, isopropanol et les diols tels que monoéthylène ou propylène glycol.

La redispersion dans le liquide non gonflant intermédiaire ou dans l'eau est facilitée par l'addition de surfactants.

Par le terme "surfactant" on entend toutes les substances à caractère amphiphile telles que les émulsifiants anioniques (alkyl-sulfate, alkyl-sulfonates, alkyl-aryl-sulfonates, dialkylsulfosuccinates, sels d'acides gras ...), cationiques (sels de cetyl-pyridinium, sels d'alkyl-benzyl ammonium, sels d'amines grasses), non ioniques (nonyl-phénols-ethoxylés, monooléate de sorbitan éthoxylé, copolymères oxyde d'éthylène-oxyde de propylène, ...) mais également des polymères solubles dans le milieu de suspension susceptibles de s'adsorber à la surface des particules. Parmi ces derniers, on peut citer l'alcool polyvinylique, les dérivés cellulosiques (hydroxyalkylcelluloses, ...), la polyvinylpyrrolidone, l'acide polyacrylique, des résines copolymères (styrène-maléique, methylvinylether maléique, acrylique-maléique), des polymères cationiques comme la polyallylamine ou bien encore des macromolécules naturelles comme la gélatine ou

5

EP 0 285 502 B1

la bovine sérum albumine.

La présente invention a également pour objet des particules composites polymère/métal calibrées, dans lesquelles le métal est totalement encapsulé dans le polymère. Le terme "calibrées" signigie de taille uniforme avec un écart type en diamètre inférieur à 5 %.

Lesdites particules composites polymère/métal de l'invention sont caractérisées en ce que :

- elles sont compactes, calibrées, présentant une taille uniforme de l'ordre de 0,1 micron à 1 mm, de préférence de l'ordre de 0,5 à 100 microns, et sont constituées :
  . d'une matrice à base d'un polymère portant des sites nucléophiles coordinables, la quantité de sites nucléophiles coordinables représentant de l'ordre de 0,5 à 30 %, de préférence de l'ordre de 3 à 30 %, du poids dudit polymère ;
  . et, encapsulées dans ladite matrice, de cristallites d'un métal à l'état de valence zéro, cristallites dont la taille est de l'ordre de $10^{-6}$ à $10^{-4}$ mm, de préférence de l'ordre de $3\ 10^{-6}$ à $3\ 10^{-5}$ mm;
- la quantité de métal encapsulé représente environ de 0,5 à 67 %, de préférence de 3 à 26 % du poids desdites particules ;
- le métal est choisi parmi les metaux dont les complexes carbonyles, organocarbonyles ou hydrocarbonés sont thermiquement instables ;
- la nature dudit polymère est telle que ce dernier est susceptible de gonfler de 0,1 à 50 fois, de préférence de 3 à 10 fois, son volume en présence d'un liquide organique solvant desdits complexes métalcarbonyles, métal organocarbonyles ou métalhydrocarbonés, le point d'ébullition dudit liquide organique étant supérieur à la température de décomposition desdits complexes.

Le métal entrant dans la composition desdites particules peut être tout métal magnétisable ou non, susceptible de former des complexes carbonyles, organocarbonyles ou hydrocarbonés décomposables par thermolyse, éventuellement sous atmosphère réductrice, jusqu'à l'état métal de valence zéro. On peut citer à titre d'exemple les métaux des groupes VIa, VIIa et VIII de la classification périodique des éléments tels que le chrome, le molybdène, le tungstène, le manganèse, le rhénium, le fer, le cobalt, le rhodium, l'iridium, le nickel, l'osmium le ruthénium...

Le polymère constituant la matrice peut être tout polymère réticulé portant des sites nucléophiles susceptibles de se coordiner aux complexes carbonyles, organocarbonyles ou hydrocarbonés dudit métal, ledit polymère devant en outre être susceptible de gonfler en présence d'un liquide organique solvant d'un desdits complexes, jusqu'à un volume tel qu'il devient accessible aux molécules dudit complexe.

Les polymères pouvant constituer la matrice des particules composites faisant l'objet de l'invention sont ceux déjà cités ci-dessus.

Un cas particulier des particules composites polymère/métal compactes calibrées faisant l'objet de l'invention sont des particules de taille uniforme de l'ordre de 0,1 micron à 1 mm, de préférence de l'ordre de 0,5 à 100 micron et constituées :

- de 99,5 à 43 % de préférence 97 à 74 %, en poids d'une matrice constituée d'un copolymère dérivé de 30 à 99 %, de préférence 50 à 95 % en poids d'au moins un monomère monoethylenique, de 0,5 à 50 %, de préférence 2 à 20 % en poids d'au moins un monomère polyethylenique réticulable et de 0,5 à 30 %, de préférence de 3 à 30 % en poids d'au moins un monomère nucléophile coordinable
- et, encapsulées dans ladite matrice, de l'ordre de 0,5 à 67 %, de préférence de l'ordre de 3 à 26 % en poids de cristallites d'un métal du groupe VIa, VIIa ou VIII de la Classification Pénodique des éléments à l'état de valence 0, la taille desdites oristallites étant de l'ordre de $10^{-6}$ à $10^{-4}$ mm, de préférence de l'ordre de $3\ 10^{-6}$ à $3\ 10^{-5}$ mm.

Lesdites particules composites calibrées peuvent être préparées selon le mode opératoire ci-dessus décrit, en mettant en oeuvre à la 1ère étape des particules calibrées de polymère portant des sites nucléophiles coordinables.

Un autre objet de l'invention consiste en des dispersions aqueuses ou latex desdites particules composites calibrées ci-dessus décrites, le taux d'extrait sec desdites dispersions (c'est-à-dire le taux de particules composites calibrées) allant de 1 à 50 % en poids, de préférence de 5 à 20 % en poids.

Lesdits latex de particules composites calibrées peuvent être préparés selon les méthodes connues par dispersion dans l'eau desdites particules composites calibrées ci-dessus décrites en présence d'émulsifiants.

D'une manière préférentielle ceux-ci sont obtenus selon le procédé de préparation de latex ci-dessus décrit, en mettant en oeuvre à la 1ère étape des particules calibrées de polymère.

Les particules composites polymère/métal telles quelles ou en dispersion aqueuse, faisant l'objet de l'invention, peuvent être utilisées comme phase solide en biologie dans les tests de diagnostic. Lorsque le métal est magnétisable, on peut tirer parti des propriétés magnétisables des particules pour les séparer facilement du milieu d'incubation et accélérer les étapes de lavage dans les tests hétérogènes du type

6

EP 0 285 502 B1

radio-immunoassays ou enzyme-immunoassays.

On peut également mentionner l'usage de ces produits en tant que support pour immobilisation d'enzymes de cellules ou d'antigènes/anticorps en biotechnologie en imagerie médicale.

D'autres applications de ces particules en dispersion aqueuse concernent les peintures, colles et encres conductrices ainsi que l'enregistrement magnétique.

Les exemples suivants sont donnés à titre indicatif et ne peuvent être considérés comme une limite du domaine et de l'esprit de l'invention.

EXEMPLE 1

A 175 g de toluène sec on ajoute 25 g de cobalt carbonyle. Cette solution est conservée à l'abri de l'air.

Dans un ballon tricol de 100 ml placé dans un bain d'huile, muni d'une arrivée d'azote, d'un dispositif d'agitation à ancre, relié à un connecteur à solvant léger et surmonté d'un réfrigérant connecté à un dispositif de mesure du monoxyde de carbone dégagé, on introduit :

- 18 g de toluène
- et 2 g de particules calibrées (diamètre moyen de 2,1 $\mu$m ± 0,1 $\mu$m) constituées d'un copolymère de styrène/divinyl benzène/4-vinylpyridine (% en poids respectifs 85/10/5), ce qui correspond à un extrait sec de 10 % en poids de copolymère réticulé dans le toluène.

On fait buller l'azote au niveau de la dispersion obtenue.

L'agitation est réglée à 100 t/mn et la température du bain d'huile est ammenée à une température suffisante (130°C) pour porter le toluène à ébullition et entrainer les dernières traces d'eau.

Après 3 heures de distillation, le réacteur est refroidi à température ambiante l'arrivée d'azote est retirée.

On introduit alors, à l'aide d'une seringue, 4,15 g de solution de cobalt carbonyle préparée précédemment (ce qui correspond à un rapport pondéral métal/particules de 0,089).

On observe aussitôt un premier dégagement gazeux de 30 ml de monoxyde de carbone correspondant au déplacement du CO (du métal carbonyle) par les motifs pyridine des particules.

On porte alors la température du bain à 130°C en 40 mn (ce qui correspond à une température interne de 110°C). Le reflux du toluène accélère le dégagement de monoxyde de carbone (56 % du volume total théorique sont dégagés dès la mise à reflux du solvant).

Après 4 h supplémentaires de chauffage à 130°C, 95 % du volume théorique de monoxyde de carbone a été recueilli ; la dispersion est passée d'un aspect rouge-orangé à un aspect noir foncé.

Le milieu réactionnel est refroidi à température ambiante.

Les particules gonflées en suspension ainsi obtenues sont attirées en présence d'un aimant. Le surnageant est parfaitement transparent et non coloré ; son analyse infra-rouge ne révèle aucune trace de cobalt carbonyle ou de complexes polynucléaires du cobalt.

Les billes sont récupérées par séparation magnétique ; le solvant résiduel est éliminé dans un évaporateur rotatif à 40°C sous 2700 Pascal puis à 60°C sous 1600 Pascal.

Sur la poudre noire obtenue, on analyse la teneur en cobalt par minéralisation et dosage des ions cobalt par absorption atomique; celle-ci est de 8,9 % (théorie : 8,9 %).

La répartition du métal au sein des particules est révélée par microscopie électronique à transmission sur des coupes ultra-minces réalisées en mélangeant les billes à une résine d'inclusion EPON 812 (résine de type epoxy commercialisée par BALZERS) et en découpant le composite obtenu à l'aide d'un ultramicrotome. On constate que le métal est distribué de façon uniforme dans tout le volume des particules composites sous forme de cristaux de $3 \ 10^{-6}$ à $15 \ 10^{-6}$ mm.

L'examen de ces mêmes particules par microscopie à balayage montre que l'imprégnation par le métal n'a pas affecté l'aspect des billes, leur surface restant parfaitement lisse et sphérique.

1 g de ces particules composites est ajouté à 50 ml d'une solution aqueuse à 1 g/l de laurylsulfate de sodium. Après 30 s de traitement aux ultra-sons, on obtient une suspension stable.

Ces particules composites peuvent être séparées du milieu en moins de 10 mn en appliquant latéralement sur le flacon contenant la dispersion un aimant de laboratoire ($8 \ 10^6$ Tesla).

Conservées sous forme de suspension aqueuse, ces particules conservent leurs propriétés magnétiques même après 6 mois de stockage.

7

EXEMPLES 2 à 10

On donne ci-après le sens des abréviations figurant aux tableaux I à IV :

S :        styrène
DVB :      divinylbenzène
MAM :     méthacrylate de méthyle
4-VP :     4-vinylpyridine
NVI :      N-vinylimidazole
DEAMS :   diethylaminométhyl styrène
AN :      acrylonitrile

On répète l'opération décrite à l'exemple 1 en mettant en oeuvre :
- des particules calibrées du copolymère défini aux tableaux I à IV, en présence du liquide gonflant ("solvant") figurant auxdits tableaux ; les quantités respectives de copolymère et de liquide gonflant figurent auxdits tableaux ("extrait sec").
- le métal carbonyl figurant aux tableaux I à IV, dissous dans ledit liquide gonflant; les quantités respectives de métal carbonyle et de copolymère figurent auxdits tableaux ("métal / particules").

Les conditions de température et de durée de l'opération de thermolyse figurent aux tableaux I à IV.

La température indiquée correspond à celle de l'intérieur du ballon et la durée correspond au temps pendant lequel le ballon est chauffé pour maintenir la température indiquée à l'intérieur du ballon (elle n'inclut pas celle nécessaire pour amener le ballon à la température indiquée).

Les caractéristiques des particules composites obtenues figurent aux tableaux I à IV.

EXEMPLE 11

On répète l'opération décrite aux exemples 1 à 10, en mettant en oeuvre des particules non calibrées de copolymère.

La nature des constituants mis en oeuvre, leurs quantités respectives, les conditions opératoires et les caractéristiques des particules composites obtenues figurent au tableau IV.

EXEMPLE 12

On répète l'opération de thermolyse décrite à l'exemple 1.

Les particules gonflées en suspension obtenues sont attirées en présence d'un aimant ; le surnageant est éliminé et remplacé par de l'éthanol (50 ml par gramme de particules).

Cette opération est répétée trois fois.

Le dernier surnageant est éliminé et remplacé par une solution aqueuse à 1 g/l de laurylsulfate de sodium (50 ml par gramme de particules). Cette opération est répétée trois fois.

Les dernières traces de toluène piégées au sein des particules sont éliminées par distillation azéotropique à 110°C pendant une heure.

On obtient ainsi un latex de particules composites dont les propriétés figurent au tableau IV.

TABLEAU I

| : EXEMPLE | : | 1 | : | 2 | : | 3 | : |
|---|---|---|---|---|---|---|---|
| : | : | | : | | : | | : |
| : Copolymère réticulé | : | | : | | : | | : |
| : - composition | : | | : | | : | | : |
| :   . S % | : | 85 | : | 65 | : | 90 | : |
| :   . DVB % | : | 10 | : | 10 | : | 5 | : |
| :   . MAM % | : | | : | | : | | : |
| :   . 4-VP % | : | 5 | : | 25 | : | 5 | : |
| :   . NVI % | : | | : | | : | | : |
| :   . DEAMS % | : | | : | | : | | : |
| :   . AN % | : | | : | | : | | : |
| : - Ø particules $\mu$m | : | 2,1 ± 0,1 | : | 2,1 ± 0,1 | : | 2,1 ± 0,1 | : |
| : - extrait sec % | : | 10 | : | 9 | : | 9 | : |
| : Solvant | : | toluène | : | toluène | : | toluène | : |
| : Métal carbonyle | : | CO2(CO)8 | : | CO2(CO)8 | : | CO2(CO)8 | : |
| :   métal/particule (poids) | : | 0,089 | : | 0,089 | : | 0,077 | : |
| : Conditions opératoires | : | | : | | : | | : |
| : - température °C | : | 110 | : | 110 | : | 133 | : |
| : - durée        h | : | 4 | : | 5 | : | 6,25 | : |
| : Particules composites | : | | : | | : | | : |
| : - Ø $\mu$m | : | 2,1 ± 0,1 | : | 2,1 ± 0,1 | : | 2,1 ± 0,1 | : |
| : - % métal | : | 8,9 | : | 8,7 | : | 7,5 | : |
| : - Ø cristallites mm x $10^{-6}$ | : | 3-15 | : | ‹ 2 | : | 3-13 | : |

TABLEAU II

| : EXEMPLE | : 4 | : 5 | : 6 | : |
|---|---|---|---|---|
| : | : | : | : | |
| : Copolymère réticulé | : | : | : | : |
| : - composition | : | : | : | : |
| :  . S % | : 90 | : 75 | : 85 | : |
| :  . DVB % | : 10 | : 10 | : 5 | : |
| :  . MAM % | : | : | : | : |
| :  . 4-VP % | : 5 | : 15 | : | : |
| :  . NVI % | : | : | : 10 | : |
| :  . DEAMS % | : | : | : | : |
| :  . AN % | : | : | : | : |
| : - Ø particules $\mu$m | : 2,1 $\pm$ 0,1 | : 2,1 $\pm$ 0,1 | : 2,1 $\pm$ 0,1 | : |
| : - extrait sec % | : 8,4 | : 11,3 | : 8,7 | : |
| : Solvant | : toluène | :chlorobenzène: | xylène | : |
| : Métal carbonyle | : CO2(CO)8 | : Fe(CO)5 | : CO2(CO)8 | : |
| :  métal/particule (poids) | : 0,178 | : 0,133 | : 0,091 | : |
| : Conditions opératoires | : | : | : | : |
| : - température °C | : 110 | : 150 | : 135 | : |
| : - durée          h | : 6,16 | : 22 | : 6,5 | : |
| : Particules composites | : | : | : | : |
| : - Ø $\mu$m | : 2,1 $\pm$ 0,1 | : 2,1 $\pm$ 0,1 | : 2,1 $\pm$ 0,1 | : |
| : - % métal | : 18 | : 13 | : 8,7 | : |
| : - Ø cristallites mm x $10^{-6}$ : | - | : - | : 3-8 | : |

TABLEAU III

| EXEMPLE | 7 | 8 | 9 |
|---|---|---|---|
| **Copolymère réticulé** | | | |
| - composition | | | |
| . S % | 85 | 85 | 60 |
| . DVB % | 10 | 5 | 10 |
| . MAM % | | | 25 |
| . 4-VP % | | | 5 |
| . NVI % | | | |
| . DEAMS % | 5 | | |
| . AN % | | 10 | . |
| - ∅ particules $\ell$ m | 2,1 ± 0,1 | 2,1 ± 0,1 | 0,95 ± 0,05 |
| - extrait sec % | 8,7 | 11,1 | 9 |
| **Solvant** | toluène | xylène | chlorobenzène |
| **Métal carbonyle** | CO2(CO)8 | Fe(CO)5 | Cr(CO)6 |
| métal/particule (poids) | 0,9 | 0,114 | 0,05 |
| **Conditions opératoires** | | | |
| - température °C | 110 | 135 | 150 |
| - durée h | 5,5 | 7,25 | 10 |
| **Particules composites** | | | |
| - ∅ $\ell$ m | 2,1 ± 0,1 | 2,1 ± 0,1 | 0,95±0,05 |
| - % métal | 9 | 11,1 | 5 |
| - ∅ cristallites mm | - | - | - |

TABLEAU IV

| : EXEMPLE | : | 10 | : | 11 | : | 12 | : |
|---|---|---|---|---|---|---|---|
| : | : | | : | | : | | : |
| : Copolymère réticulé | : | | : | | : | | : |
| : - composition | : | | : | | : | | : |
| : . S % | : | 85 | : | 85 | : | 85 | : |
| : . DVB % | : | 10 | : | 10 | : | 10 | : |
| : . MAM % | : | | : | | : | | : |
| : . 4-VP % | : | 5 | : | 5 | : | 5 | : |
| : . NVI % | : | | : | | : | | : |
| : . DEAMS % | : | | : | | : | | : |
| : . AN % | : | | : | | : | | : |
| : - Ø particules Cm | : | 9,7 ± 0,5 | : | 52 ± 23 | : | 2,1 ± 1 | : |
| : - extrait sec % | : | 9 | : | 9 | : | 10 | : |
| : Solvant | : | toluène | : | toluène | : | toluène | : |
| : Métal carbonyle | : | CO2(CO)8 | : | CO2(CO)8 | : | CO2(CO)8 | : |
| : métal/particule (poids) | : | 0,05 | : | 0,05 | : | 0,089 | : |
| : Conditions opératoires | : | | : | | : | | : |
| : - température °C | : | 110 | : | 110 | : | 110 | : |
| : - durée h | : | 4 | : | 4 | : | 4 | : |
| : Particules composites | : | | : | | : | | : |
| : - Ø Cm | : | 9,7 ± 0,5 | : | 52 ± 23 | : | 2,1 ± 0,1 | : |
| : - % métal | : | 4,9 | : | 5 | : | 8,9 | : |
| : - Ø cristallites mm x $10^{-6}$ : | | - | : | 3 - 15 | : | 3 - 15 | : |

## Revendications

**Revendications pour les Etats contractants suivants : AT, BE, CH, DE, FR, GB, GR, IT, LI, LU, NL, SE**

1. Procédé de préparation de particules compactes composites polymère/métal comprenant les étapes suivantes :

   a) on disperse dans un liquide organique, solvant de complexes métalcarbonyles, organocarbonyles ou hydrocarbonés décomposables thermiquement à une température inférieure au point d'ébullition dudit liquide organique, des particules anhydres de polymère insolubles dans ledit liquide organique et portant des sites nucléophiles coordinables auxdits complexes, la taille desdites particules étant de l'ordre de 0,1 micron à 1 mm, la nature dudit polymère constituant lesdites particules étant telle que lesdites particules sont susceptibles de gonfler de 0,1 à 50 fois, leur volume en présence dudit liquide organique ;

   b) on introduit dans la dispersion de particules de polymère gonflées ainsi obtenue, un complexe métal carbonyle, organocarbonyle ou hydrocarboné soluble dans ledit liquide organique, selon un rapport pondéral métal dudit complexe/polymère de l'ordre de 0,5/100 à 200/100, et on effectue une opération de thermolyse dudit complexe, cette opération ayant lieu sous atmosphère réductrice lorsque ledit complexe est un complexe métal hydrocarboné

c) on élimine ledit liquide organique gonflant

d) éventuellement on redisperse lesdites particules composites compactes ainsi obtenues dans un liquide non gonflant dudit polymère.

2. Procédé selon la revendication 1 caractérisé en ce qu'après élimination du liquide organique gonflant à l'étape c), les particules composites obtenues sont lavées à l'aide d'un liquide non gonflant intermédiaire miscible avec le liquide organique gonflant et avec l'eau, puis séparées dudit liquide non gonflant intermédiaire et dispersées dans de l'eau par lavage à l'eau.

3. Particules composites polymère/métal de taille uniforme de l'ordre de 0,1 micron à 1 mm caractérisées en ce que :
- elles sont compactes et sont constituées :
  . d'une matrice à base d'un polymère portant des sites nucléophiles coordinables, la quantité de sites nucléophiles coordinables représentant de l'ordre de 0,5 à 30 % du poids dudit polymère ;
  . et, totalement encapsulées dans ladite matrice, de cristallites d'un métal à l'état de valence zéro, cristallites dont la taille est de l'ordre de $10^{-6}$ à $10^{-4}$ mm
- la quantité de métal encapsulé représente environ de 0,5 à 67 %, du poids desdites particules ;
- le métal est choisi parmi les métaux dont les complexes carbonyles, organocarbonyles ou hydrocarbonés sont thermiquement instables ;
- la nature dudit polymère est telle que ce dernier est susceptible de gonfler de 0,1 à 50 fois son volume en présence d'un liquide organique non solvant dudit polymère mais solvant desdits complexes métalcarbonyles, métal organocarbonyles ou métalhydrocarbonés, le point d'ébullition dudit liquide organique étant supérieur à la température de décomposition desdits complexes.

4. Particules composites selon la revendication 3 caractérisées en ce que la quantité de sites nucléophiles de la matrice est de l'ordre de 3 à 30 % du poids de polymère.

5. Particules composites polymère/métal de taille uniforme de l'ordre de 0,1 à 1 mm caractérisées en ce qu'elles sont compactes et constituées :
- de 99,5 à 43 % en poids d'une matrice constituée d'un copolymère dérivé de 30 à 99 % en poids d'au moins un monomère monoethylenique, de 0,5 à 50 % en poids d'au moins un monomère polyethylenique réticulable et de 0,5 à 30 % en poids d'au moins un monomère nucléophile coordinable
- et, totalement encapsulées dans ladite matrice, de l'ordre de 0,5 à 67 % en poids de cristallites d'un métal du groupe VIa, VIIa ou VIII de la Classification Périodique des éléments à l'état de valence 0, la taille desdites cristallites étant de l'ordre de $10^{-6}$ à $10^{-4}$ mm.

6. Particules composites calibrées selon l'une quelconque des revendications 3 à 5 caracterisées en ce qu'elles sont calibrées c'est-à-dire qu'elles présentent une taille uniforme de l'ordre de 0,5 à 100 microns, et un écart type en diamètre inférieur à 5 %.

7. Particules composites selon l'une quelconque des revendications 3 à 6 caractérisées en ce qu'elles sont constituées de 97 à 74 % en poids de matrice et de 3 à 26 % en poids de métal.

8. Particules composites selon l'une quelconque des revendications 3 à 7 caractérisées en ce que la taille des cristallites de métal est de l'ordre de $3 \cdot 10^{-6}$ à $3 \cdot 10^{-5}$ mm.

9. Dispersions aqueuses de particules composites, caractérisées en ce qu'elles comprennent de 1 à 50 % en poids des particules composites calibrées faisant l'objet de l'une quelconque des revendications 3 à 8.

10. Procédé de préparation des particules composites faisant l'objet de la revendication 3, caractérisé en ce qu'il comprend les étapes suivantes :
a) on disperse dans un liquide organique, solvant de complexes métalcarbonyles, organocarbonyles ou hydrocarbonés décomposables thermiquement à une température inférieure au point d'ébullition dudit liquide organique, des particules anhydres et calibrées de polymère insoluble dans ledit liquide organique et portant des sites nucléophiles coordinables auxdits complexes, la taille desdites particules étant uniforme et de l'ordre de 0,1 micron à 1 mm, la nature dudit polymère constituant lesdites particules étant telle que lesdites particules sont susceptibles de gonfler de 0,1 à 50 fois leur volume en présence dudit liquide organique ;

b) on introduit dans la dispersion de particules de polymère gonflées ainsi obtenue, un complexe métal carbonyle, organocarbonyle ou hydrocarboné soluble dans ledit liquide organique, selon un rapport pondéral métal dudit complexe/polymère de l'ordre de 0,5/100 à 200/100, et on effectue une opération de thermolyse dudit complexe, cette opération ayant lieu sous atmosphère réductrice lorsque ledit complexe est un complexe métal hydrocarboné

c) on élimine ledit liquide organique gonflant

d) éventuellement on redisperse lesdites particules composites compactes ainsi obtenues dans un liquide non gonflant dudit polymère.

**11.** Procédé de préparation de dispersions aqueuses des particules composites faisant l'objet de la revendication 3, caractérisé en ce que :

a) on disperse dans un liquide organique, solvant de complexes métalcarbonyles, organocarbonyles ou hydrocarbonés décomposables thermiquement à une température inférieure au point d'ébullition dudit liquide organique, des particules anhydres et calibrées de polymère insoluble dans ledit liquide organique et portant des sites nucléophiles coordinables auxdits complexes, la taille desdites particules étant uniforme et de l'ordre de 0,1 micron à 1 mm, la nature dudit polymère constituant lesdites particules étant telle que lesdites particules sont susceptibles de gonfler de 0,1 à 50 fois leur volume en présence dudit liquide organique ;

b) on introduit dans la dispersion de particules de polymère gonflées ainsi obtenue, un complexe métal carbonyle, organocarbonyle ou hydrocarboné soluble dans ledit liquide organique, selon un rapport pondéral métal dudit complexe/polymère de l'ordre de 0,5/100 à 200/100, et on effectue une opération de thermolyse dudit complexe, cette opération ayant lieu sous atmosphère réductrice lorsque ledit complexe est un complexe métal hydrocarboné

c) on élimine ledit liquide organique gonflant

d) on lave les particules composites obtenues à l'aide d'un liquide non gonflant intermédiaire miscible avec ledit liquide organique gonflant et avec de l'eau

e) on elimine ledit liquide non gonflant

f) on disperse dans l'eau les particules composites obtenues, par lavage à l'eau jusqu'à obtenir une concentration dans l'eau des particules de l'ordre de 1 à 50% en poids.

**12.** Procédé selon l'une quelconque des revendications 1, 2, 10 ou 11 caractérisé en ce qu'à l'étape a) la taille des particules de polymère est de l'ordre de 0,5 à 100 microns.

**13.** Procédé selon l'une quelconque des revendications 1, 2, 10 - 12 caractérisé en ce qu'à l'étape a) les particules de polymère sont susceptibles de gonfler de 3 à 10 fois leur volume en présence dudit liquide organique.

**14.** Procédé selon l'une quelconque des revendications 1, 2, 10 - 13 caractérisé en ce qu'à l'étape b) le rapport pondéral métal dudit complexe/polymère est de l'ordre de 3/100 à 35/100.

**15.** Procédé selon l'une quelconque des revendications 1, 2, 10 - 14 caractérisé en ce que ledit polymère est constitué de :
- 30 à 99 % en poids d'au moins un monomère monoéthylénique, 0,5 à 50 % en poids d'au moins un monomère polyéthylénique réticulable, et 0,5 à 30 % en poids d'au moins un monomère nucléophile coordinable.

**16.** Procédé selon la revendication 15, caractérisé en ce que ledit polymère est constitué de :
- 50 à 95 % en poids d'au moins un monomère monoéthylénique
- 2 à 20 % en poids d'au moins un monomère polyéthylénique réticulable
- 3 à 30 % en poids d'au moins un monomère nucléophile coordinable.

**17.** Procédé selon la revendication 15 ou 16 caractérisé en ce que ledit monomère monoéthylénique est du styrène, un dérivé du styrène, un ester ou un amide de l'acide acrylique ou méthacrylique, de l'acide acrylique ou méthacrylique, un diacide monoéthylénique, de la vinylpyrrolidone, un ester vinylique, du chlorure de vinyle ou de vinylidène.

**18.** Procédé selon l'une quelconque uus revendications 15 à 17 caractérisé en ce que ledit monomère polyéthylénique est du divinylbenzène, un dérivé du divinylbenzène, un diène conjugué, un dérivé polyallylique, un acrylate ou méthacrylate de polyol, du méthylène-bis (acrylamide), de l'acide bis (acrylamido)acétique.

**19.** Procédé selon l'une quelconque des revendications 15 à 18 caractérisé en ce que le monomère nucléophile cuordinable est une vinylpyridine, un di(éthyl)amino-alkylacrylate ou méthacrylate, un di (éthyl) amino-alkylacrylamide or méthacrylamide, de l'allylamine, de l'éthylène imine, de l'acrylonitrile ou du méthacrylonitrile, du 1-vinylimidazole, un dialkylaminométhylstyrène.

**20.** Procédé selon l'une quelconque des revendications 1, 2, 10 - 19 caractérisé en ce que ledit liquide organique gonflant est un dérivé aromatique, éventuellement chloré, un hydrocarbure aliphatique ou cycloaliphatique, un dialkyl éther, un alcool, un ester, de point d'ébullition supérieur à la température de décomposition dudit complexe.

**21.** Procédé selon l'une quelconque des revendications 1, 2, 10 - 20, caractérisé en ce que la concentration de particules de polymère dans le liquide organique gonflant à l'étape a) va de 1 à 35 % en poids.

**22.** Procédé selon l'une quelconque des revendications 1, 2, 10 - 21 caractérisé en ce que ledit complexe est un complexe carbonyle organocarbonyle de type aliphatique, cycloaliphatique ou aromatique, hydrocarboné de type aliphatique, cycloaliphatique des métaux des groupes VIa, VIIa et VIII de la Classification périodique des éléments.

**23.** Procédé selon la revendication 22 caractérisé en ce que lesdits complexes sont de formule $Cr(CO)_6$ ; $Mo(CO)_6$ ; $W(CO)_6$ ; $(Mn)_2 (CO)_{10}$ ; $(Re)_2(CO)_{10}$ ; $Fe(CO)_5$ ; $(Fe)_2(CO)_9$ ; $(Fe)_3(CO)_{12}$ ; $(CO)_2(CO)_8$ ; $(CO)_4(CO)_{12}$ ; $Ni(CO)_4$ ; $(Rh)_6(CO)_{12}$ ; $(Rh)_4(CO)_{12}$ ; $(Ir)_4(CO)_{12}$ ; $(Os)_3(CO)_{12}$ ; $(Ru)_3(CO)_{12}$ - (methylcyclopentadienyl)$Mn(CO)_3$ ; (cyclopentadienyl)$Mn(CO)_3$ ; (cyclopentadienyl) $Co(CO)_2$ ; (cyclopentadienyl)$Re(CO)_3$ ; (di-$^5$-cyclopentadienyl)Ni ou Fe

**24.** Procédé selon l'une quelconque des revendications 1, 2, 10 - 23 caractérisé en ce que l'opération de thermolyse est réalisée à une température allant de 80 à 200 °C.

**25.** Procédé selon la revendication 24 caractérisé en ce que l'opération de thermolyse est réalisée à une température voisine de celle d'ébullition du liquide organique gonflant.

**26.** Utilisation des particules compactes composites polymère/métal telles quelles ou en dispersion aqueuse obtenues selon le procédé de l'une quelconque des revendications 1, 2, 10 - 25 en biologie.

**27.** Utilisation des particules compactes composites polymère/métal telles quelles ou en dispersions aqueuses faisant l'objet des revendications 3 à 9 comme phase solide en biologie dans les tests de diagnostic.

**Revendications pour l'Etat contractant suivant : ES**

**1.** Procédé de préparation de particules compactes composites polymère/métal comprenant les étapes suivantes :

a) on disperse dans un liquide organique, solvant de complexes métalcarbonyles, organocarbonyles ou hydrocarbonés décomposables thermiquement à une température inférieure au point d'ébullition dudit liquide organique, des particules anhydres de polymère insolubles dans ledit liquide organique et portant des sites nucléophiles coordinables auxdits complexes, la taille desdites particules étant de l'ordre de 0,1 micron à 1 mm, la nature dudit polymère constituant lesdites particules étant telle que lesdites particules sont susceptibles de gonfler de 0,1 à 50 fois, leur volume en présence dudit liquide organique ;

b) on introduit dans la dispersion de particules de polymère gonflées ainsi obtenue, un complexe métal carbonyle, organocarbonyle ou hydrocarboné soluble dans ledit liquide organique, selon un rapport pondéral métal dudit complexe/polymère de l'ordre de 0,5/100 à 200/100, et on effectue une

EP 0 285 502 B1

opération de thermolyse dudit complexe, cette opération ayant lieu sous atmosphère réductrice lorsque ledit complexe est un complexe métal hydrocarboné
c) on élimine ledit liquide organique gonflant
d) éventuellement on redisperse lesdites particules composites compactes ainsi obtenues dans un liquide non gonflant dudit polymère.

2. Procédé selon la revendication 1 caractérisé en ce qu'après élimination du liquide organique gonflant à l'étape c), les particules composites obtenues sont lavées à l'aide d'un liquide non gonflant intermédiaire miscible avec le liquide organique gonflant et avec l'eau, puis séparées dudit liquide non gonflant intermédiaire et dispersées dans de l'eau par lavage à l'eau.

3. Procédé de préparation de particules composites polymère/métal de taille uniforme de l'ordre de 0,1 micron à 1 mm, caractérisé en ce que lesdites particules présentent les caractéristiques suivantes :
   - elles sont compactes et, sont constituées :
     . d'une matrice à base d'un polymère portant des sites nucléophiles coordinables, la quantité de sites nucléophiles coordinables représentant de l'ordre de 0,5 à 30 % du poids dudit polymère ;
     . et, totalement encapsulées dans ladite matrice, de cristallites d'un métal à l'état de valence zéro, cristallites dont la taille est de l'ordre de $10^{-6}$ à $10^{-4}$ mm
   - la quantité de métal encapsulé représente environ de 0,5 à 67 %, du poids desdites particules ;
   - le métal est choisi parmi les métaux dont les complexes carbonyles, organocarbonyles ou hydrocarbonés sont thermiquement instables ;
   - la nature dudit polymère est telle que ce dernier est susceptible de gonfler de 0,1 à 50 fois son volume en présence d'un liquide organique non solvant dudit polymère mais solvant desdits complexes métalcarbonyles, métal organocarbonyles ou métalhydrocarbonés, le point d'ébullition dudit liquide organique étant supérieur à la température de décomposition desdits complexes,
   et en ce que ledit procédé comprend les étapes suivantes :
   a) on disperse dans un liquide organique, solvant de complexes métalcarbonyles, organocarbonyles ou hydrocarbonés décomposables thermiquement à une température inférieure au point d'ébullition dudit liquide organique, des particules anhydres et calibrées de polymère insoluble dans ledit liquide organique et portant des sites nucléophiles coordinables auxdits complexes, la taille desdites particules étant uniforme et de l'ordre de 0,1 micron à 1 mm, la nature dudit polymère constituant lesdites particules étant telle que lesdites particules sont susceptibles de gonfler de 0,1 à 50 fois leur volume en présence dudit liquide organique ;
   b) on introduit dans la dispersion de particules de polymère gonflées ainsi obtenue, un complexe métal carbonyle, organocarbonyle ou hydrocarboné soluble dans ledit liquide organique, selon un rapport pondéral métal dudit complexe/polymère de l'ordre de 0,5/100 à 200/100, et on effectue une opération de thermolyse dudit complexe, cette opération ayant lieu sous atmosphère réductrice lorsque ledit complexe est un complexe métal hydrocarboné
   c) on élimine ledit liquide organique gonflant
   d) éventuellement on redisperse lesdites particules composites compactes ainsi obtenues dans un liquide non gonflant dudit polymère.

4. Procédé selon la revendication 3 caractérisées en ce que la quantité de sites nucléophiles de la matrice est de l'ordre de 3 à 30 % du poids de polymère.

5. Procédé de préparation de particules composites polymère/métal de taille uniforme de l'ordre de 0,1 à 1 mm caractérisées en ce qu'elles sont compactes et constituées :
   - de 99,5 à 43 % en poids d'une matrice constituée d'un copolymère dérivé de 30 à 99 % en poids d'au moins un monomère monoethylenique, de 0,5 à 50 % en poids d'au moins un monomère polyethylenique réticulable et de 0,5 à 30 % en poids d'au moins un monomère nucléophile coordinable
   - et, totalement encapsulées dans ladite matrice, de l'ordre de 0,5 à 67 % en poids de cristallites d'un métal du groupe VIa, VIIa ou VIII de la Classification Périodique des éléments à l'état de valence 0, la taille desdites cristallites étant de l'ordre de $10^{-6}$ à $10^{-4}$ mm,
   en ce que ledit procédé présente les étapes suivantes :
   a) on disperse dans un liquide organique, solvant de complexes métalcarbonyles, organocarbonyles ou hydrocarbonés décomposables thermiquement à une température inférieure au point d'ébullition

16

EP 0 285 502 B1

dudit liquide organique, des particules anhydres et calibrées de polymère insoluble dans ledit liquide organique et portant des sites nucléophiles coordinables auxdits complexes, la taille desdites particules étant uniforme et de l'ordre de 0,1 micron à 1 mm, la nature dudit polymère constituant lesdites particules étant telle que lesdites particules sont susceptibles de gonfler de 0,1 à 50 fois leur volume en présence dudit liquide organique ;

b) on introduit dans la dispersion de particules de polymère gonflées ainsi obtenue, un complexe métal carbonyle, organocarbonyle ou hydrocarboné soluble dans ledit liquide organique, selon un rapport pondéral métal dudit complexe/polymère de l'ordre de 0,5/100 à 200/100, et on effectue une opération de thermolyse dudit complexe, cette opération ayant lieu sous atmosphère réductrice lorsque ledit complexe est un complexe métal hydrocarboné

c) on élimine ledit liquide organique gonflant

d) éventuellement on redisperse lesdites particules composites compactes ainsi obtenues dans un liquide non gonflant dudit polymère.

6. Procédé selon l'une quelconque des revendications 3 à 5 caractérisées en ce qu'elles sont calibrées c'est-à-dire qu'elles présentent une taille uniforme de l'ordre de 0,5 à 100 microns, et un écart type en diamètre inférieur à 5 %.

7. Procédé selon l'une quelconque des revendications 3 à 6 caractérisées en ce qu'elles sont constituées de 97 à 74 % en poids de matrice et de 3 à 26 % en poids de métal.

8. Procédé selon l'une quelconque des revendications 3 à 7 caractérisées en ce que la taille des cristallites de métal est de l'ordre de $3\ 10^{-6}$ à $3\ 10^{-5}$ mm.

9. Procédé selon l'une des revendications 3 à 8, caractérisé en ce que les particules sont sous la forme de dispersions aqueuses comprenant de 1 à 50 % en poids des particules composites calibrées.

10. Procédé de préparation de dispersions aqueuses des particules composites définie à la revendication 3, caractérisé en ce que :

a) on disperse dans un liquide organique, solvant de complexes métalcarbonyles, organocarbonyles ou hydrocarbonés décomposables thermiquement à une température inférieure au point d'ébullition dudit liquide organique des particules anhydres et calibrées de polymère insoluble dans ledit liquide organique et portant des sites nucléophiles coordinables auxdits complexes, la taille desdites particules étant uniforme et de l'ordre de 0,1 micron à 1 mm, la nature dudit polymère constituant lesdites particules étant telle que lesdites particules sont susceptibles de gonfler de 0,1 à 50 fois leur volume en présence dudit liquide organique ;

b) on introduit dans la dispersion de particules de polymère gonflées ainsi obtenue, un complexe métal carbonyle, organocarbonyle ou hydrocarboné soluble dans ledit liquide organique, selon un rapport pondéral métal dudit complexe/polymère de l'ordre de 0,5/100 à 200/100, et on effectue une opération de thermolyse dudit complexe, cette opération ayant lieu sous atmosphère réductrice lorsque ledit complexe est un complexe métal hydrocarboné

c) on élimine ledit liquide organique gonflant

d) on lave les particules composites obtenues à l'aide d'un liquide non gonflant intermédiaire miscible avec ledit liquide organique gonflant et avec de l'eau

e) on élimine ledit liquide non gonflant

f) on disperse dans l'eau les particules composites obtenues, par lavage à l'eau jusqu'à obtenir une concentration dans l'eau des particules de l'ordre de 1 à 50% en poids.

11. Procédé selon l'une quelconque des revendications précédentes caractérisé en ce qu'à l'étape a) la taille des particules de polymère est de l'ordre de 0,5 à 100 microns.

12. Procédé selon l'une quelconque des revendications précédentes caractérisé en ce qu'à l'étape a) les particules de polymère sont susceptibles de gonfler de 3 à 10 fois leur volume en présence dudit liquide organique.

13. Procédé selon l'une quelconque des revendications précédentes caractérisé en ce qu'à l'étape b) le rapport pondéral métal dudit complexe/polymère est de l'ordre de 3/100 à 35/100.

17

14. Procédé selon l'une quelconque des revendications précédentes caractérisé en ce que ledit polymère est constitué de :
- 30 à 99 % en poids d'au moins un monomère monoéthylénique, 0,5 à 50 % en poids d'au moins un monomère polyéthylénique réticulable, et 0,5 à 30 % en poids d'au moins un monomère nucléophile coordinable.

15. Procédé selon la revendication 14, caractérisé en ce que ledit polymère est constitué de :
- 50 à 95 % en poids d'au moins un monomère monoéthylénique
- 2 à 20 % en poids d'au moins un monomère polyéthylénique réticulable
- 3 à 30 % en poids d'au moins un monomère nucléophile coordinable.

16. Procédé selon la revendication 14 ou 15 caractérisé en ce que ledit monomère monoéthylénique est du styrène, un dérivé du styrène, un ester ou un amide de l'acide acrylique ou méthacrylique, de l'acide acrylique ou méthacrylique, un diacide monoéthylénique, de la vinylpyrrolidone, un ester vinylique, du chlorure de vinyle ou de vinylidène.

17. Procédé selon l'une quelconque des revendications 14 à 16 caractérisé en ce que ledit monomère polyéthylénique est du divinylbenzène, un dérivé du divinylbenzène, un diène conjugué, un dérivé polyallylique, un acrylate ou méthacrylate de polyol, du méthylène-bis (acrylamide), de l'acide bis (acrylamido)acétique.

18. Procédé selon l'une quelconque des revendications 14 à 17 caractérisé en ce que le monomère nucléophile coordinable est une vinylpyridine, un di(éthyl)amino-alkylacrylate ou méthacrylate, un di (éthyl) amino-alkylacrylamide or méthacrylamide, de l'allylamine, de l'éthylène imine, de l'acrylonitrile ou du méthacrylonitrile, du 1-vinylimidazole, un dialkylaminométhylstyrène.

19. Procédé selon l'une quelconque des revendications précédentes caractérisé en ce que ledit liquide organique gonflant est un dérivé aromatique, éventuellement chloré, un hydrocarbure aliphatique ou cycloaliphatique, un dialkyl éther, un alcool, un ester, de point d'ébullition supérieur à la température de décomposition dudit complexe.

20. Procédé selon l'une quelconque des revendications précédentes caractérisé en ce que la concentration de particules de polymère dans le liquide organique gonflant à l'étape a) va de 1 à 35 % en poids.

21. Procédé selon l'une quelconque des revendications précédentes caractérisé en ce que ledit complexe est un complexe carbonyle organocarbonyle de type aliphatique, cycloaliphatique ou aromatique, hydrocarboné de type aliphatique, cycloaliphatique des métaux des groupes VIa, VIIa et VIII de la Classification périodique des éléments.

22. Procédé selon la revendication 21 caractérisé en ce que lesdits complexes sont de formule $Cr(CO)_6$ ; $Mo(CO)_6$ ; $W(CO)_6$ $(Mn)_2 (CO)_{10}$ ; $(Re)_2(CO)_{10}$ ; $Fe(CO)_5$ ; $(Fe)_2(CO)_9$ ; $(Fe)_3(CO)_{12}$ ; $(CO)_2(CO)_8$ ; $(Co)_4(CO)_{12}$ ; $Ni(CO)_4$ ; $(Rh)_6(CO)_{12}$ ; $(Rh)_4(CO)_{12}$ ; $(Ir)_4(CO)_{12}$ ; $(Os)_3(CO)_{12}$ ; $(Ru)_3(CO)_{12}$ -(methylcyclopentadienyl)$Mn(CO)_3$ ; (cyclopentadienyl)$Mn(CO)_3$ ; (cyclopentadienyl) $Co(CO)_2$ ; (cyclopentadienyl)$Re(CO)_3$ (di-[5]-cyclopentadienyl)Ni ou Fe

23. Procédé selon l'une quelconque des revendications précédentes caractérisé en ce que l'opération de thermolyse est réalisée à une température allant de 80 à 200°C.

24. Procédé selon la revendication 23 caractérisé en ce que l'opération de thermolyse est réalisée à une température voisine de celle d'ébullition du liquide organique gonflant.

25. Utilisation des particules compactes composites polymère/métal telles quelles ou en dispersion aqueuse, obtenues selon le procédé de l'une quelconque des revendications précédentes en biologie.

26. Utilisation des particules compactes composites polymère/métal telles quelles ou en dispersions aqueuses obtenues selon l'une des revendications 3 à 24 comme phase solide en biologie dans les tests de diagnostic.

**Claims**

**Claims for the following Contracting States : AT, BE, CH, DE, FR, GB, GR, IT, LI, LU, NL, SE**

1. Process for preparing compact composite polymer/metal particles comprising the following stages:

   a) in an organic liquid which is a solvent for metal carbonyl, organocarbonyl or hydrocarbon complexes which are thermally decomposable at a temperature lower than the boiling point of the said organic liquid, anhydrous polymer particles insoluble in the said organic liquid and carrying nucleophilic sites coordinatable with the said complexes are dispersed, the size of the said particles being around 0.1 microns to 1 mm, the nature of the said polymer constituting the said particles being such that the said particles are able to swell by 0.1 to 50 times their volume in the presence of the said organic liquid;

   b) a metal carbonyl, organocarbonyl or hydrocarbon complex soluble in the said organic liquid is introduced into the dispersion of swollen polymer particles thus obtained, in a ratio by weight between the metal of the said complex and the polymer of around 0.5:100 to 200:100, and thermolysis of the said complex is carried out, this operation taking place in a reducing atmosphere when the said complex is a metal hydrocarbon complex;

   c) the said organic liquid causing swelling is eliminated;

   d) optionally the said compact composite particles thus obtained are redispersed in a liquid which does not cause swelling of the said polymer.

2. Process according to Claim 1, characterised in that, after elimination of the organic liquid causing swelling at stage c), the composite particles obtained are washed by means of an intermediate liquid not causing swelling which is miscible with the organic liquid causing swelling and with water, and then separated from the said intermediate liquid not causing swelling and dispersed in water by washing with water.

3. Composite polymer/metal particles of uniform size of around 0.1 microns to 1 mm, characterised in that:

   - they are compact and consist of:

     . a polymer-based matrix carrying coordinatable nucleophilic sites, the quantity of coordinatable nucleophilic sites representing around 0.5 to 30% of the weight of the said polymer;

     . and, completely encapsulated in the said matrix, crystallites of a metal in the null valency state, crystallites whose size is of the order of $10^{-6}$ to $10^{-4}$ mm;

   - the quantity of encapsulated metal represents approximately 0.5 to 67% of the weight of the said particles;

   - the metal is chosen from amongst those metals whose carbonyl, organocarbonyl or hydrocarbon complexes are thermally unstable;

   - the nature of the said polymer is such that the latter is able to swell by 0.1 to 50 times its volume in the presence of an organic liquid which is not a solvent for the said polymer but is a solvent for the said metal carbonyl, organocarbonyl or hydrocarbon complexes, the boiling point of the said organic liquid being higher than the decomposition temperature of the said complexes.

4. Composite particles according to Claim 3, characterised in that the quantity of nucleophilic sites of the matrix is around 3 to 30% of the weight of the polymer.

5. Composite polymer/metal particles of uniform size of around 0.1 to 1 mm, characterised in that they are compact and consist of:

   - from 99.5 to 43% by weight of a matrix consisting of a copolymer derived to the extent of 30 to 99% by weight from at least one monoethyl monomer, 0.5 to 50% by weight from at least one cross-linkable polyethyl monomer and 0.5 to 30% by weight from at least one coordinatable nucleophilic monomer,

   - and, completely encapsulated in the said matrix, around 0.5 to 67% by weight of crystallites of a metal in groups VIa, VIIa or VIII in the periodic table of elements in the null valency state, the size of the said crystallites being of the order of $10^{-6}$ to $10^{-4}$ mm.

6. Calibrated composite particles according to any one of Claims 3 to 5, characterised in that they are calibrated, that is to say they have a uniform size of around 0.5 to 100 microns, and a standard deviation in diameter of less than 5%.

**7.** Composite particles according to any one of Claims 3 to 6, characterised in that they consist of 97 to 74% by weight of matrix and 3 to 26% by weight of metal.

**8.** Composite particles according to any one of Claims 3 to 7, characterised in that the size of the metal crystallites is of the order of $3 \times 10^{-6}$ to $3 \times 10^{-5}$ mm.

**9.** Aqueous dispersions of composite particles, characterised in that they comprise from 1 to 50% by weight of the calibrated composite particles forming the object of any one of Claims 3 to 8.

**10.** Process for preparing composite particles forming the object of Claim 3, characterised in that it comprises the following stages:

a) in an organic liquid which is a solvent for metal carbonyl, organocarbonyl or hydrocarbon complexes which are thermally decomposable at a temperature lower than the boiling point of the said organic liquid, anhydrous calibrated particles of polymer insoluble in the said organic liquid and carrying nucleophilic sites coordinatable with the said complexes are dispersed, the size of the said particles being uniform and around 0.1 microns to 1 mm, the nature of the said polymer constituting the said particles being such that the said particles are able to swell by 0.1 to 50 times their volume in the presence of the said organic liquid;

b) a metal carbonyl, organocarbonyl or hydrocarbon complex soluble in the said organic liquid is introduced into the dispersion of swollen polymer particles thus obtained, in a ratio by weight between the metal of the said complex and the polymer of around 0.5:100 to 200:100, and thermolysis of the said complex is carried out, this operation taking place in a reducing atmosphere when the said complex is a metal hydrocarbon complex;

c) the said organic liquid causing swelling is eliminated;

d) optionally the said compact composite particles thus obtained are redispersed in a liquid which does not cause swelling of the said polymer.

**11.** Process for preparing aqueous dispersions of the composite particles forming the object of Claim 3, characterised in that:

a) in an organic liquid which is a solvent for metal carbonyl, organocarbonyl or hydrocarbon complexes which are thermally decomposable at a temperature lower than the boiling point of the said organic liquid, anhydrous calibrated particles of polymer insoluble in the said organic liquid and carrying nucleophilic sites coordinatable with the said complexes are dispersed, the size of the said particles being uniform and around 0.1 microns to 1 mm, the nature of the said polymer constituting the said particles being such that the said particles are able to swell by 0.1 to 50 times their volume in the presence of the said organic liquid;

b) a carbonyl metal, organocarbonyl or hydrocarbon complex soluble in the said organic liquid is introduced into the dispersion of swollen polymer particles thus obtained, in a ratio by weight between the metal of the said complex and the polymer of around 0.5:100 to 200:100, and thermolysis of the said complex is carried out, this operation taking place in a reducing atmosphere when the said complex is a metal hydrocarbon metal;

c) the said organic liquid causing swelling is eliminated;

d) the composite particles obtained are washed by means of an intermediate liquid which does not cause swelling and is miscible with the said organic liquid causing swelling and with water;

e) the said liquid not causing swelling is eliminated;

f) the composite particles obtained are dispersed in water, by washing with water until a concentration of the particles in water of around 1 to 50% by weight is obtained.

**12.** Process according to any one of Claims 1, 2, 10 or 11, characterised in that at stage a) the size of the polymer particles is around 0.5 to 100 microns.

**13.** Process according to any one of Claims 1, 2, 10-12, characterised in that at stage a) the polymer particles are able to swell by 3 to 10 times their volume in the presence of the said organic liquid.

**14.** Process according to any one of Claims 1, 2, 10-13, characterised in that at stage b) the ratio by weight of metal in the said complex to polymer is around 3:100 to 35:100.

**15.** Process according to any one of Claims 1, 2, 10-14, characterised in that the said polymer consists of:

20

- 30 to 99% by weight of at least one monoethylene monomer, 0.5 to 50% by weight of at least one cross-linkable polyethylene monomer, and 0.5 to 30% by weight of at least one coordinatable nucleophilic monomer.

**16.** Process according to Claim 15, characterised in that the said polymer consists of:
- 50 to 95% by weight of at least one monoethylene monomer
- 2 to 20% by weight of at least one cross-linkable polyethylene monomer
- 3 to 30% by weight of at least one coordinatable nucleophilic monomer.

**17.** Process according to Claim 15 or 16, characterised in that the said monoethylene monomer is styrene, a derivative of styrene, an ester or an amide of acrylic or methacrylic acid, acrylic or methacrylic acid, a monoethylene diacid, vinylpyrrolidone, a vinyl ester, vinyl chloride or vinylidene.

**18.** Process according to any one of Claims 15 to 17, characterised in that the said polyethylene monomer is divinylbenzene, a derivative of divinylbenzene, a related diene, a polyallyl derivative, a polyol acrylate or methacrylate, methylene-bis(acrylamide) or bis(acrylamido) acetic acid.

**19.** Process according to any one of Claims 15 to 18, characterised in that the coordinatable nucleophilic monomer is a vinylpyridine, a di(ethyl)amino alkylacrylate or methacrylate, a di(ethyl)amino alkyl acrylamide or methacrylamide, allyl amine, ethyl amine, acrylonitrile or methacrylonitrile, 1-vinyl-imidazole, or a dialkylaminomethylstyrene.

**20.** Process according to any one of Claims 1, 2, 10-19, characterised in that the said organic liquid causing swelling is an aromatic derivative, optionally chlorinated, an aliphatic or cycloaliphatic hydrocarbon, a dialkyl ether, an alcohol or an ester, with a boiling point higher than the decomposition temperature of the said complex.

**21.** Process according to any one of Claims 1, 2, 10-20, characterised in that the concentration of polymer particles in the organic liquid causing swelling at stage a) ranges from 1 to 35% by weight.

**22.** Process according to any one of Claims 1, 2, 10-21, characterised in that the said complex is a carbonyl complex, an organocarbonyl complex of the aliphatic, cycloaliphatic or aromatic type, or a hydrocarbon complex of the aliphatic or cycloaliphatic type of the metals in groups VIa, VIIa and VIII in the periodic table of elements.

**23.** Process according to Claim 22, characterised in that the said complexes are of the formula $Cr(CO)_6$; $Mo(CO)_6$; $W(CO)_6$; $(Mn)_2(CO)_{10}$; $(Re)_2(CO)_{10}$; $Fe(CO)_5$; $(Fe)_2(CO)_9$; $(Fe)_3(CO)_{12}$; $(CO)_2(CO)_8$; $(Co)_4-(CO)_{12}$; $Ni(CO)_4$; $(Rh)_6(CO)_{12}$; $(Rh)_4(CO)_{12}$; $(Ir)_4(CO)_{12}$; $(Os)_3(CO)_{12}$;$(Ru)_3(CO)_{12}$; (methylcyclopentadienyl)$Mn(CO)_3$; (cyclopentadienyl)$Mn(CO)_3$; (cyclopentadienyl)$Co(CO)_2$;(cyclopentadienyl)$Re(CO)_3$; (di-$^5$-cyclopentadienyl)Ni or Fe.

**24.** Process according to any one of Claims 1, 2, 10-23, characterised in that,the thermolysis is carried out at a temperature ranging from 80 to 200°C.

**25.** Process according to Claim 24, characterised in that the thermolysis is carried out at a temperature close to the boiling point of the organic liquid causing swelling.

**26.** Use in biology of the compact composite polymer/metal particles as they are or in an aqueous dispersion obtained according to the process of any one of Claims 1, 2, 10-25.

**27.** Use of the compact composite polymer/metal particles as they are or in aqueous dispersions forming the object of Claims 3 to 9 as a solid phase in biology in diagnostic tests.

**Claims for the following Contracting State : ES**

**1.** Process for preparing compact composite polymer/metal particles comprising the following stages:
    a) in an organic liquid which is a solvent for metal carbonyl, organocarbonyl or hydrocarbon complexes which are thermally decomposable at a temperature lower than the boiling point of the said organic liquid, anhydrous polymer particles insoluble in the said organic liquid and carrying nucleophilic sites coordinatable with the said complexes are dispersed, the size of the said particles being around 0.1 microns to 1 mm, the nature of the said polymer constituting the said particles being such that the said particles are able to swell by 0.1 to 50 times their volume in the presence of the said organic liquid;

b) a metal carbonyl, organocarbonyl or hydrocarbon complex soluble in the said organic liquid is introduced into the dispersion of swollen polymer particles thus obtained, in a ratio by weight between the metal of the said complex and the polymer of around 0.5:100 to 200:100, and thermolysis of the said complex is carried out, this operation taking place in a reducing atmosphere when the said complex is a metal hydrocarbon complex;

c) the said organic liquid causing swelling is eliminated;

d) optionally the said compact composite particles thus obtained are redispersed in a liquid which does not cause swelling of the said polymer.

2. Process according to Claim 1, characterised in that, after elimination of the organic liquid causing swelling at stage c), the composite particles obtained are washed by means of an intermediate liquid not causing swelling which is miscible with the organic liquid causing swelling and with water, and then separated from the said intermediate liquid not causing swelling and dispersed in water by washing with water.

3. Process for preparing composite polymer/metal particles of uniform side of around 0.1 microns to 1 mm, characterised in that the said particles have the following characteristics:

- they are compact and consist of:
  . a polymer-based matrix carrying coordinatable nucleophilic sites, the quantity of coordinatable nucleophilic sites representing around 0.5 to 30% of the weight of the said polymer;
  . and, completely encapsulated in the said matrix, crystallites of a metal in the null valency state, crystallites whose size is of the order of $10^{-6}$ to $10^{-4}$ mm;
- the quantity of encapsulated metal represents approximately 0.5 to 67% of the weight of the said particles;
- the metal is chosen from amongst those metals whose carbonyl, organocarbonyl or hydrocarbon complexes are thermally unstable;
- the nature of the said polymer is such that the latter is able to swell by 0.1 to 50 times its volume in the presence of an organic liquid which is not a solvent for the said polymer but is a solvent for the said metal carbonyl, organocarbonyl or hydrocarbon complexes, the boiling point of the said organic liquid being higher than the decomposition temperature of the said complexes,

and in that the said process comprises the following stages:

a) in an organic liquid which is a solvent for metal carbonyl, organocarbonyl or hydrocarbon complexes which are thermally decomposable at a temperature lower than the boiling point of the said organic liquid, anhydrous calibrated particles of polymer insoluble in the said organic liquid and carrying nucleophilic sites coordinatable with the said complexes are dispersed, the size of the said particles being uniform and around 0.1 microns to 1 mm, the nature of the said polymer constituting the said particles being such that the said particles are able to swell by 0.1 to 50 times their volume in the presence of the said organic liquid;

b) a metal carbonyl, organocarbonyl or hydrocarbon complex soluble in the said organic liquid is introduced into the dispersion of swollen polymer particles thus obtained, in a ratio by weight between the metal of the said complex and the polymer of around 0.5:100 to 200:100, and thermolysis of the said complex is carried out, this operation taking place in a reducing atmosphere when the said complex is a metal hydrocarbon complex;

c) the said organic liquid causing swelling is eliminated;

d) optionally the said compact composite particles thus obtained are redispersed in a liquid which does not cause swelling of the said polymer.

4. Process according to Claim 3, characterised in that the quantity of nucleophilic sites of the matrix is around 3 to 30% of the weight of the polymer.

5. Process for preparing composite polymer/metal particles of uniform size of around 0.1 to 1 mm, characterised in that they are compact and consist of:

EP 0 285 502 B1

- from 99.5 to 43% by weight of a matrix consisting of a copolymer derived to the extent of 30 to 99% by weight from at least one monoethyl monomer, 0.5 to 50% by weight from at least one cross-linkable polyethyl monomer and 0.5 to 30% by weight from at least one coordinatable nucleophilic monomer,
- and, completely encapsulated in the said matrix, around 0.5 to 67% by weight of crystallites of a metal in groups VIa, VIIa or VIII in the periodic table of elements in the null valency state, the size of the said crystallites being of the order of $10^{-6}$ to $10^{-4}$ mm,

in that the said process has the following stages:

a) in an organic liquid which is a solvent for metal carbonyl, organocarbonyl or hydrocarbon complexes which are thermally decomposable at a temperature lower than the boiling point of the said organic liquid, anhydrous calibrated particles of polymer insoluble in the said organic liquid and carrying nucleophilic sites coordinatable with the said complexes are dispersed, the size of the said particles being uniform and around 0.1 microns to 1 mm, the nature of the said polymer constituting the said particles being such that the said particles are able to swell by 0.1 to 50 times their volume in the presence of the said organic liquid;

b) a metal carbonyl, organocarbonyl or hydrocarbon complex soluble in the said organic liquid is introduced into the dispersion of swollen polymer particles thus obtained, in a ratio by weight between the metal of the said complex and the polymer of around 0.5:100 to 200:100, and thermolysis of the said complex is carried out, this operation taking place in a reducing atmosphere when the said complex is a metal hydrocarbon complex;

c) the said organic liquid causing swelling is eliminated;

d) optionally the said compact composite particles thus obtained are redispersed in a liquid which does not cause swelling of the said polymer.

6. Process according to any one of Claims 3 to 5, characterised in that they are calibrated, that is to say they have a uniform size of around 0.5 to 100 microns, and a standard deviation in diameter of less than 5%.

7. Process according to any one of Claims 3 to 6, characterised in that they consist of 97 to 74% by weight of matrix and 3 to 26% by weight of metal.

8. Process according to any one of Claims 3 to 7, characterised in that the size of the metal crystallites is of the order of $3 \times 10^{-6}$ to $3 \times 10^{-5}$ mm.

9. Process according to one of claims 3 to 8, characterised in that the particles are in the form of aqueous dispersions comprising from 1 to 50% by weight of the calibrated composite particles.

10. Process for preparing aqueous dispersions of the composite particles defined in Claim 3, characterised in that:

a) in an organic liquid which is a solvent for metal carbonyl, organocarbonyl or hydrocarbon complexes which are thermally decomposable at a temperature lower than the boiling point of the said organic liquid, anhydrous calibrated particles of polymer insoluble in the said organic liquid and carrying nucleophilic sites coordinatable with the said complexes are dispersed, the size of the said particles being uniform and around 0.1 microns to 1 mm, the nature of the said polymer constituting the said particles being such that the said particles are able to swell by 0.1 to 50 times their volume in the presence of the said organic liquid;

b) a carbonyl metal, organocarbonyl or hydrocarbon complex soluble in the said organic liquid is introduced into the dispersion of swollen polymer particles thus obtained, in a ratio by weight between the metal of the said complex and the polymer of around 0.5:100 to 200:100, and thermolysis of the said complex is carried out, this operation taking place in a reducing atmosphere when the said complex is a metal hydrocarbon metal;

c) the said organic liquid causing swelling is eliminated;

d) the composite particles obtained are washed by means of an intermediate liquid which does not cause swelling and is miscible with the said organic liquid causing swelling and with water;

e) the said liquid not causing swelling is eliminated;

f) the composite particles obtained are dispersed in water, by washing with water until a concentration of the particles in water of around 1 to 50% by weight is obtained.

23

11. Process according to any one of the preceding claims, characterised in that at stage a) the size of the polymer particles is around 0.5 to 100 microns.

12. Process according to any one of the preceding claims, characterised in that at stage a) the polymer particles are able to swell by 3 to 10 times their volume in the presence of the said organic liquid.

13. Process according to any one of the preceding claims, characterised in that at stage b) the ratio by weight of metal in the said complex to polymer is around 3:100 to 35:100.

14. Process according to any one of the preceding claims, characterised in that the said polymer consists of:
    - 30 to 99% by weight of at least one monoethylene monomer, 0.5 to 50% by weight of at least one cross-linkable polyethylene monomer, and 0.5 to 30% by weight of at least one coordinatable nucleophilic monomer.

15. Process according to Claim 14, characterised in that the said polymer consists of:
    - 50 to 95% by weight of at least one monoethylene monomer
    - 2 to 20% by weight of at least one cross-linkable polyethylene monomer
    - 3 to 30% by weight of at least one coordinatable nucleophilic monomer.

16. Process according to Claim 14 or 15, characterised in that the said monoethylene monomer is styrene, a derivative of styrene, an ester or an amide of acrylic or methacrylic acid, acrylic or methacrylic acid, a monoethylene diacid, vinylpyrrolidone, a vinyl ester, vinyl chloride or vinylidene.

17. Process according to any one of Claims 14 to 16, characterised in that the said polyethylene monomer is divinylbenzene, a derivative of divinylbenzene, a related diene, a polyallyl derivative, a polyol acrylate or methacrylate, methylene-bis(acrylamide) or bis(acrylamido) acetic acid.

18. Process according to any one of Claims 14 to 17, characterised in that the coordinatable nucleophilic monomer is a vinylpyridine, a di(ethyl)amino alkylacrylate or methacrylate, a di(ethyl)amino alkyl acrylamide or methacrylamide, allyl amine, ethyl amine, acrylonitrile or methacrylonitrile, 1-vinyl-imidazole, or a dialkylaminomethylstyrene.

19. Process according to any one of the preceding claims, characterised in that the said organic liquid causing swelling is an aromatic derivative, optionally chlorinated, an aliphatic or cycloaliphatic hydrocarbon, a dialkyl ether, an alcohol or an ester, with a boiling point higher than the decomposition temperature of the said complex.

20. Process according to any one of the preceding claims, characterised in that the concentration of polymer particles in the organic liquid causing swelling at stage a) ranges from 1 to 35% by weight.

21. Process according to any one of the preceding claims, characterised in that the said complex is a carbonyl complex, an organocarbonyl complex of the aliphatic, cycloaliphatic or aromatic type, or a hydrocarbon complex of the aliphatic or cycloaliphatic type of the metals in groups VIa, VIIa and VIII in the periodic table of elements.

22. Process according to Claim 21, characterised in that the said complexes are of the formula $Cr(CO)_6$; $Mo(CO)_6$; $W(CO)_6$; $(Mn)_2(Co)_{10}$; $(Re)_2(CO)_{10}$; $Fe(CO)_5$; $(Fe)_2(CO)_9$; $(Fe)_3(CO)_{12}$; $(CO)_2(CO)_8$; $(Co)_4(CO)_{12}$; $Ni(CO)_4$; $(Rh)_6(CO)_{12}$; $(Rh)_4(CO)_{12}$; $(Ir)_4(Co)_{12}$; $(Os)_3(CO)_{12}$; $(Ru)_3(CO)_{12}$; (methylcyclopentadienyl)-$Mn(CO)_3$; (cyclopentadienyl)$Mn(CO)_3$; (cyclopentadienyl)$Co(CO)_2$; (cyclopentadienyl)$Re(CO)_3$; (di-5-cyclopentadienyl)Ni or Fe.

23. Process according to any one of the preceding claims, characterised in that the thermolysis is carried out at a temperature ranging from 80 to 200°C.

24. Process according to Claim 23, characterised in that the thermolysis is carried out at a temperature close to the boiling point of the organic liquid causing swelling.

**25.** Use in biology of the compact composite polymer/metal particles as they are or in an aqueous dispersion obtained according to the process of any one of the preceding claims.

**26.** Use of the compact composite polymer/metal particles as they are or in aqueous dispersions obtained according to one of Claims 3 to 24 as a solid phase in biology in diagnostic tests.

**Patentansprüche**
**Patentansprüche für folgende Vertragsstaaten : AT, BE, CH, DE, FR, GB, GR, IT, LI, LU, NL, SE**

**1.** Verfahren zur Herstellung von zusammengesetzten kompakten Teilchen aus Polymer/Metall, welches folgende Schritte umfaßt:

a) man dispergiert in einer organischen Flüssigkeit, Lösungsmittel für Metallcarbonyl-, Organocarbonyl- oder Kohlenwasserstoffkomplexe, thermisch zersetzbar bei einer Temperatur niedriger als der Siedepunkt der organischen Flüssigkeit, wasserfreie Polymerteilchen, die in der genannten organischen Flüssigkeit unlöslich sind und welche nucleophile, an die genannten Komplexe koordinierbare Stellen tragen, wobei die Größe der genannten Teilchen in dem Bereich von 0,1 Mikron bis 1 mm liegt, wobei die Beschaffenheit des Polymeren, aus welchem die Teilchen bestehen, eine solche ist, daß die Partikel geeignet sind, in Gegenwart der organischen Flüssigkeit auf das 0,1- bis 50-fache ihres Volumens aufgebläht zu werden;

b) man führt in die Dispersion der so erhaltenen aufgeblähten Polymerteilchen einen Metallcarbonyl, Organocarbonyl- oder Kohlenwasserstoffkomplex, der in der organischen Flüssigkeit löslich ist, ein, gemäß einem Gewichtsverhältnis von Metallkomplex/Polymer in dem Bereich von 0,5/100 bis 200/100, und man bewirkt eine Thermolysereaktion des Komplexes, wobei diese Reaktion unter Reduktionsatmosphare durchgeführt wird, wenn der Komplex ein Metallkohlenwasserstoffkomplex ist,

c) man entfernt die organische Aufblähflüssigkeit,

d) man suspendiert gegebenenfalls die so erhaltenen zusammengesetzten kompakten Teilchen erneut in einer das Polymer nicht-aufblähenden Flüssigkeit.

**2.** Verfahren nach Anspruch 1, dadurch gekennzeichnet, daß nach dem Entfernen der organischen Aufblähflüssigkeit in Schritt c) die erhaltenen zusammengesetzten Teilchen mit Hilfe einer nichtaufblähenden Flüssigkeit, intermediär mit der organischen Aufblähflüssigkeit und mit Wasser mischbar, gewaschen werden, dann von der nicht-aufblähenden intermediären Flüssigkeit getrennt und in Wasser zum Waschen in Wasser dispergiert werden.

**3.** Zusammengesetzte Teilchen aus Polymer/Metall mit einer einheitlichen Größe in dem Bereich von 0,1 Mikron bis 1 mm, dadurch gekennzeichnet, daß:

- sie kompakt sind und bestehen aus:
  . einer Matrix auf Polymerbasis, welches nucleophile, koordinierbare Stellen trägt, wobei die Menge der nucleophilen, koordinierbaren Stellen in dem Bereich von 0,5 bis 30 Gewichts-% des Polymeren vorliegt;
  . und vollständig in dieser Matrix eingekapselten Kristalliten aus Metall im Valenzzustand null, wobei die Kristallite eine Größe in dem Bereich von $10^{-6}$ bis $10^{-4}$ mm haben,
- die Menge des eingekapselten Metalls etwa 0,5 bis 67 Gewichts-% der Teilchen beträgt;
- das Metall ausgewählt ist aus den Metallen, deren Carbonyl-, Organocarbonyl- oder Kohlenwasserstoffkomplexe thermisch instabil sind;
- die Beschaffenheit des Polymers eine solche ist, daß letzteres geeignet ist, in Gegenwart einer organischen Flüssigkeit, die nicht Lösungsmittel für das Polymer ist, aber Lösungsmittel für die Metallcarbonyl-, Metallorganocarbonyl- oder Metallkohlenwasserstoffkomplexe ist, aufgebläht zu werden, wobei der Siedepunkt der organischen Flüssigkeit über der Zersetzungstemperatur der Komplexe liegt.

**4.** Zusammengesetzte Teilchen nach Anspruch 3, dadurch gekennzeichnet, daß die Menge der nucleophilen Stellen der Matrix in dem Bereich von 3 bis 30 Gewichts-% des Polymeren liegt.

**5.** Zusammengesetzte Polymer/Metallteilchen mit einheitlicher Größe in dem Bereich von 0,1 bis 1 mm, dadurch gekennzeichnet, daß sie kompakt sind und bestehen aus:

- 99,5 bis 43 Gewichts-% einer Matrix, bestehend aus einem Copolymer erhalten aus 30 bis 99 Gewichts-% mindestens eines monoethylenischen Monomeren, 0,5 bis 50 Gewichts-% minde

stens eines polyethylenisch vernetzbaren Monomeren und 0,5 bis 30 Gewichts-% mindestens eines nucleophilen, koordinierbaren Monomeren
- und vollständig in die Matrix eingekapselte Kristallite aus einem Metall aus der Gruppe VIa, VIIa oder VIII des Periodensystems der Elemente mit dem Valenzzustand 0, in dem Bereich von 0,5 bis 67 Gewichts-%, wobei die Größe dieser Kristallite in dem Bereich von $10^{-6}$ bis $10^{-4}$ mm liegt.

6. Zusammengesetzte Teilchen nach einem der Ansprüche 3 bis 5, dadurch gekennzeichnet, daß sie kalibriert sind, daß heißt, daß sie eine einheitliche Größe in dem Bereich von 0,5 bis 100 Mikron und eine Standardabweichung im Durchmesser unter 5 % aufweisen.

7. Zusammengesetzte Teilchen nach einem der Ansprüche 3 bis 6, dadurch gekennzeichnet, daß sie zu 97 bis 74 Gewichts-% aus der Matrix und zu 3 bis 26 Gewichts-% aus Metall bestehen.

8. Zusammengesetzte Teilchen nach einem der Ansprüche 3 bis 7, dadurch gekennzeichnet, daß die Größe der Kristallite des Metalls in dem Bereich von $3 \times 10^{-6}$ bis $3 \times 10^{-5}$ mm ist.

9. Wäßrige Dispersionen aus zusammengesetzten Teilchen, dadurch gekennzeichnet, daß sie 1 bis 50 Gewichts-% der kalibrierten zusammengesetzten Teilchen, die der Gegenstand von einem der Ansprüche 3 bis 8 sind, enthalten.

10. Verfahren zur Herstellung von zusammengesetzten Teilchen, die Gegenstand von Anspruch 3 sind, dadurch gekennzeichnet, daß es die folgenden Schritte umfaßt:
a) man dispergiert in einer organischen Flüssigkeit, Lösungsmittel für Metallcarbonyl-, Organocarbonyl- oder Kohlenwasserstoffkomplexe, thermisch zersetzbar bei einer Temperatur niedriger als der Siedepunkt der organischen Flüssigkeit, wasserfreie und kalibrierte Polymerteilchen, die in der genannten organischen Flüssigkeit unlöslich sind und welche nucleophile, an die genannten Komplexe koordinierbare Stellen tragen, wobei die Größe der genannten Teilchen in dem Bereich von 0,1 Mikron bis 1 mm liegt, wobei die Beschaffenheit des Polymeren, aus welchem die Teilchen bestehen, eine solche ist, daß die Partikel geeignet sind, in Gegenwart der organischen Flüssigkeit auf das 0,1- bis 50-fache ihres Volumens aufgebläht zu werden;
b) man führt in die Dispersion der so erhaltenen aufgeblähten Polymerteilchen einen Metallcarbonyl-, Organocarbonyl- oder Kohlenwasserstoffkomplex, der in der organischen Flüssigkeit löslich ist, ein, gemäß einem Gewichtsverhältnis von Metallkomplex/Polymer in dem Bereich von 0,5/100 bis 200/100, und man bewirkt eine Thermolysereaktion des Komplexes, wobei diese Reaktion unter Reduktionsatmosphäre durchgeführt wird, wenn der Komplex ein Metallkohlenwasserstoffkomplex ist,
c) man entfernt die organische Aufblähflüssigkeit,
d) man suspendiert gegebenenfalls die so erhaltenen zusammengesetzten kompakten Teilchen erneut in einer das Polymer nicht-aufblähenden Flüssigkeit.

11. Verfahren zur Herstellung von wäßrigen Dispersionen aus zusammengesetzten Teilchen, die Gegenstand des Anspruchs 3 sind, dadurch gekennzeichnet, daß:
a) man in einer organischen Flüssigkeit, die Lösungsmittel ist für Metallcarbonyl-, Organocarbonyl- oder Kohlenwasserstoffkomplexe, welche bei einer Temperatur niedriger als der Siedepunkt der organischen Flüssigkeit thermisch zersetzbar sind, wasserfreie und kalibrierte Polymerteilchen dispergiert, die in der genannten organischen Flüssigkeit unlöslich sind und welche nucleophile, an die genannten Komplexe koordinierbare Stellen tragen, wobei die Größe der genannten Teilchen in dem Bereich von 0,1 Mikron bis 1 mm liegt, die wobei Beschaffenheit des Polymeren, aus welchem die Teilchen bestehen, eine solche ist, daß die Partikel geeignet sind, in Gegenwart der organischen Flüssigkeit auf das 0,1- bis 50-fache ihres Volumens aufgebläht zu werden;
b) man in die Dispersion der so erhaltenen aufgeblähten Polymerteilchen einen Metallcarbonyl-, Organocarbonyl- oder Kohlenwasserstoffkomplex, der in der organischen Flüssigkeit löslich ist, einführt, gemäß einem Gewichtsverhältnis von Metallkomplex/Polymer in dem Bereich von 0,5/100 bis 200/100, und man eine Thermolysereaktion des Komplexes bewirkt, wobei diese Reaktion unter Reduktionsatmosphäre durchgeführt wird, wenn der Komplex ein Metallkohlenwasserstoffkomplex ist,
c) man die organische Aufblähflüssigkeit entfernt,

d) man die erhaltenen zusammengesetzten Teilchen mit einer nicht-aufblähenden, intermediär mit der organischen Aufblähflüssigkeit und mit Wasser mischbaren Flüssigkeit wäscht,

e) man die nicht-aufblähende Flüssigkeit entfernt,

f) man die erhaltenen zusammengesetzten Teilchen in Wasser dispergiert, durch Waschen mit Wasser bis in Wasser eine Konzentration der Teilchen in dem Bereich von 1 bis 50 Gewichts-% erhalten wird.

12. Verfahren nach einem der einem der Ansprüche 1, 2, 10 oder 11, dadurch gekennzeichnet, daß in Schritt a) die Größe der Polymerteilchen in dem Bereich von 0,5 bis 100 Mikronen liegt.

13. Verfahren nach einem der Ansprüche 1, 2, 10-12, dadurch gekennzeichnet, daß in Schritt a) die Polymerteilchen geeignet sind, in Gegenwart der organischen Flüssigkeit auf das 3- bis 10-fache ihres Volumens aufgebläht zu werden.

14. Verfahren nach einem der Ansprüche 1, 2, 10-13, dadurch gekennzeichnet, daß in Schritt b) das Gewichtsverhältnis Metallkomplex/Polymer in dem Bereich von 3/100 bis 35/100 liegt.

15. Verfahren nach einem der Ansprüche 1, 2, 10 - 14, dadurch gekennzeichnet, daß das Polymer besteht aus:
   - 30 bis 99 Gewichts-% mindestens eines monoethylenischen Monomeren, 0,5 bis 50 Gewichts-% mindestens eines polyethylenisch vernetzbaren Monomeren und 0,5 bis 30 Gewichts-% mindestens eines nucleophil koordinierbaren Monomeren.

16. Verfahren nach Anspruch 15, dadurch gekennzeichnet, daß das Polymer besteht aus:
   - 50 bis 95 Gewichts-% mindestens eines monoethylenischen Monomeren,
   - 2 bis 20 Gewichts-% mindestens eines Polyethylenisch vernetzbaren Monomeren
   - 3 bis 30 Gewichts-% mindestens eines nucleophil koordinierbaren Monomeren.

17. Verfahren nach Anspruch 15 oder 16, dadurch gekennzeichnet, daß das monoethylenische Monomere Styrol, ein Styrolderivat, ein Acrylsäure- oder Methacrylsäureester oder -amid, Acryl- oder Methacrylsäure, eine monoethylenische Disäure, ein Vinylpyrrolidon, ein Vinylester, Vinyl- oder Vinylidenchlorid ist.

18. Verfahren nach einem der Ansprüche 15 bis 17, dadurch gekennzeichnet, daß das polyethylenische Monomere Divinylbenzol, ein Divinylbenzolderivat, ein konjugiertes Dien, ein Polyallylderivat, ein Polyolacrylat oder -methacrylat, Methylen-bis(acrylamid), Bis(acrylamid)essigsäure ist.

19. Verfahren nach einem der Ansprüche 15 bis 18, dadurch gekennzeichnet, daß das nucleophil koordinierbare Monomer Vinylpyridin, ein Di(ethyl)aminoalkylacrylat oder -methacrylat, ein Di(ethyl)-aminoalkylacrylamid oder -methacrylamid, Allylamin, Ethylenimin, Acrylnitril oder Methacrylnitril, 1-Vinylimidazol, ein Dialkylaminomethylstyrol ist.

20. Verfahren nach einem der Ansprüche 1, 2, 10 - 9, dadurch gekennzeichnet, daß die organische Aufblähflüssigkeit ein aromatisches Derivat ist, gegebenenfalls chloriert, ein aliphatischer oder cycloaliphatischer Kohlenwasserstoff, ein Dialkylether, ein Alkohol, ein Ester, wobei der Siedepunkt über der Zersetzungstemperatur des Komplexes liegt.

21. Verfahren nach einem der Ansprüche 1, 2, 10 - 20, dadurch gekennzeichnet, daß die Konzentration der Polymerteilchen in der organischen Aufblähflüssigkeit in Schritt a) von 1 bis 35 Gewichts-% reicht.

22. Verfahren nach einem der Ansprüche 1, 2, 10 - 21, dadurch gekennzeichnet, daß der Carbonylkomplex Organocarbonyl vom aliphatischen, cycloaliphatischen oder aromatischen Typ, Kohlenwasserstoff vom aliphatischen, cycloaliphatischen Typ mit Metallen der Gruppen VIa, VIIa und VIII des Periodensystems der Elemente ist.

23. Verfahren nach Anspruch 22, dadurch gekennzeichnet, daß die Komplexe die Formeln haben $Cr(CO)_6$; $Mo(CO)_6$; $W(CO)_6$; $(Mn)_2(CO)_{10}$; $(Re)_2(CO)_{10}$; $Fe(CO)_5$; $(Fe)_2(CO)_9$; $(Fe)_3(CO)_{12}$; $(Co)_2(CO)_8$; $(Co)_4(CO)_{12}$; $Ni(CO)_4$; $(Rh)_6(Co)_{12}$; $(Rh)_4(CO)_{12}$; $(Ir)_4(CO)_{12}$; $(Os)_3(CO)_{12}$; $(Ru)_3(CO)_{12}$ (Methylcyclopentadienyl)-

Mn(CO)$_3$; (Cyclopentadienyl)Mn(CO)$_3$; (Cyclopentadienyl)Co(CO)$_2$;(Cyclopentadienyl)Re(CO)$_3$ (Bi-$\eta^5$-cyclopentadienyl)Ni oder -Fe.

24. Verfahren nach einem der Ansprüche 1, 2, 10 - 23, dadurch gekennzeichnet, daß die Thermolysereaktion bei einer Temperatur von 80 bis 200°C durchgeführt wird.

25. Verfahren nach Anspruch 24, dadurch gekennzeichnet, daß die Thermolysereaktion bei einer Temperatur in der Nähe des Siedepunktes der organischen Aufblähflüssigkeit durchgeführt wird.

26. Verwendung der kompakten zusammengesetzten Polymer/Metallteilchen als solche oder in einer wäßrigen Dispersion, erhalten nach dem Verfahren nach einem der Ansprüche 1, 2, 10-25, in der Biologie.

27. Verwendung der kompakten zusammengesetzten Polymer/Metallteilchen als solche oder in wäßrigen Dispersionen, welche Gegenstand der Ansprüche 3 bis 9 sind, als feste Phase in der Biologie in diagnostischen Tests.

**Patentansprüche für folgenden Vertragsstaat : ES**

1. Verfahren zur Herstellung von zusammengesetzten kompakten Teilchen aus Polymer/Metall, welches folgende Schritte umfaßt:
    a) man dispergiert in einer organischen Flüssigkeit, Lösungsmittel für Metallcarbonyl-, Organocarbonyl- oder Kohlenwasserstoffkomplexe, thermisch zersetzbar bei einer Temperatur niedriger als der Siedepunkt der organischen Flüssigkeit, wasserfreie Polymerteilchen, die in der genannten organischen Flüssigkeit unlöslich sind und welche nucleophile, an die genannten Komplexe koordinierbare Stellen tragen, wobei die Größe der genannten Teilchen in dem Bereich von 0,1 Mikron bis 1 mm liegt, wobei die Beschaffenheit des Polymeren, aus welchem die Teilchen bestehen, eine solche ist, daß die Partikel geeignet sind, in Gegenwart der organischen Flüssigkeit auf das 0,1- bis 50-fache ihres Volumens aufgebläht zu werden;
    b) man führt in die Dispersion der so erhaltenen aufgeblähten Polymerteilchen einen Metallcarbonyl-, Organocarbonyl- oder Kohlenwasserstoffkomplex, der in der organischen Flüssigkeit löslich ist, ein, gemäß einem Gewichtsverhältnis von Metallkomplex/Polymer in dem Bereich von 0,5/100 bis 200/100, und man bewirkt eine Thermolysereaktion des Komplexes, wobei diese Reaktion unter Reduktionsatmosphäre durchgeführt wird, wenn der Komplex ein Metallkohlenwasserstoffkomplex ist,
    c) man entfernt die organische Aufblähflüssigkeit,
    d) man suspendiert gegebenenfalls die so erhaltenen zusammengesetzten kompakten Teilchen erneut in einer das Polymer nicht-aufblähenden Flüssigkeit.

2. Verfahren nach Anspruch 1, dadurch gekennzeichnet, daß nach dem Entfernen der organischen Aufblähflüssigkeit in Schritt c) die erhaltenen zusammengesetzten Teilchen mit Hilfe einer nicht-aufblähenden Flüssigkeit, intermediär mit der organischen Aufblähflüssigkeit und mit Wasser mischbar, gewaschen werden, dann von der nicht-aufblähenden intermediären Flüssigkeit getrennt und in Wasser zum Waschen in Wasser dispergiert werden.

3. Verfahren zur Herstellung von zusammengesetzten Teilchen aus Polymer/Metall mit einer einheitlichen Größe in dem Bereich von 0,1 Mikron bis 1 mm, dadurch gekennzeichnet, daß sie die folgenden Eigenschaften aufweisen:
    - sie kompakt sind und bestehen aus:
        . einer Matrix auf Polymerbasis, welches nucleophile, koordinierbare Stellen trägt, wobei die Menge der nucleophilen, koordinierbaren Stellen in dem Bereich von 0,5 bis 30 Gewichts-% des Polymeren vorliegt;
        . und vollständig in dieser Matrix eingekapselten Kristalliten aus Metall im Valenzzustand null, wobei die Kristallite eine Größe in dem Bereich von $10^{-6}$ bis $10^{-4}$ mm haben,
    - die Menge des eingekapselten Metalls etwa 0,5 bis 67 Gewichts-% der Teilchen beträgt;
    - das Metall ausgewählt ist aus den Metallen, deren Carbonyl-, Organocarbonyl- oder Kohlenwasserstoffkomplexe thermisch instabil sind;

28

- die Beschaffenheit des Polymers eine solche ist, daß letzteres geeignet ist, in Gegenwart einer organischen Flüssigkeit, die nicht Lösungsmittel für das Polymer ist, aber Lösungsmittel für die Metallcarbonyl-, Metallorganocarbonyl- oder Metallkohlenwasserstoffkomplexe ist, aufgebläht zu werden, wobei der Siedepunkt der organischen Flüssigkeit über der Zersetzungstemperatur der Komplexe liegt

und dadurch, daß das Verfahren die folgenden Schritte umfaßt:

a) man dispergiert in einer organischen Flüssigkeit, Lösungsmittel für Metallcarbonyl-, Organocarbonyl- oder Kohlenwasserstoffkomplexe, thermisch zersetzbar bei einer Temperatur niedriger als der Siedepunkt der organischen Flüssigkeit, wasserfreie und kalibrierte Polymerteilchen, die in der genannten organischen Flüssigkeit unlöslich sind und welche nucleophile, an die genannten Komplexe koordinierbare Stellen tragen, wobei die Größe der genannten Teilchen in dem Bereich von 0,1 Mikron bis 1 mm liegt, wobei die Beschaffenheit des Polymeren, aus welchem die Teilchen bestehen, eine solche ist, daß die Partikel geeignet sind, in Gegenwart der organischen Flüssigkeit auf das 0,1- bis 50-fache ihres Volumens aufgebläht zu werden;

b) man führt in die Dispersion der so erhaltenen aufgeblähten Polymerteilchen einen Metallcarbonyl-, Organocarbonyl- oder Kohlenwasserstoffkomplex, der in der organischen Flüssigkeit löslich ist, ein, gemäß einem Gewichtsverhältnis von Metallkomplex/Polymer in dem Bereich von 0,5/100 bis 200/100, und man bewirkt eine Thermolysereaktion des Komplexes, wobei diese Reaktion unter Reduktionsatmosphäre durchgeführt wird, wenn der Komplex ein Metallkohlenwasserstoffkomplex ist,

c) man entfernt die organische Aufblähflüssigkeit,

d) man suspendiert gegebenenfalls die so erhaltenen Zusammengesetzten kompakten Teilchen erneut in einer das Polymer nicht-aufblähenden Flüssigkeit.

4. Verfahren nach Anspruch 3, dadurch gekennzeichnet, daß die Menge der nucleophilen Stellen der Matrix in dem Bereich von 3 bis 30 Gewichts-% des Polymeren liegt.

5. Verfahren zur Herstellung von zusammengesetzte Polymer/Metallteilchen mit einheitlicher Größe in dem Bereich von 0,1 bis 1 mm, dadurch gekennzeichnet, daß sie kompakt sind und bestehen aus:

- 99,5 bis 43 Gewichts-% einer Matrix, bestehend aus einem Copolymer erhalten aus 30 bis 99 Gewichts-% mindestens eines monoethylenischen Monomeren, 0,5 bis 50 Gewichts-% mindestens eines polyethylenisch vernetzbaren Monomeren und 0,5 bis 30 Gewichts-% mindestens eines nucleophilen, koordinierbaren Monomeren

- und vollständig in die Matrix eingekapselte Kristallite aus einem Metall aus der Gruppe VIa, VIIa oder VIII des Periodensystems der Elemente mit dem Valenzzustand 0, in dem Bereich von 0,5 bis 67 Gewichts-%, wobei die Größe dieser Kristallite in dem Bereich von $10^{-6}$ bis $10^{-4}$ mm liegt,

dadurch gekennzeichnet, daß die folgenden Schritte umfaßt:

a) man dispergiert in einer organischen Flüssigkeit, Lösungsmittel für Metallcarbonyl-, Organocarbonyl- oder Kohlenwasserstoffkomplexe, thermisch zersetzbar bei einer Temperatur niedriger als der Siedepunkt der organischen Flüssigkeit, wasserfreie und kalibrierte Polymerteilchen, die in der genannten organischen Flüssigkeit unlöslich sind und welche nucleophile, an die genannten Komplexe koordinierbare stellen tragen, wobei die Größe der genannten Teilchen in dem Bereich von 0,1 Mikron bis 1 mm liegt, wobei die Beschaffenheit des Polymeren, aus welchem die Teilchen bestehen, eine solche ist, daß die Partikel geeignet sind, in Gegenwart der organischen Flüssigkeit auf das 0,1- bis 50-fache ihres Volumens aufgebläht zu werden;

b) man führt in die Dispersion der so erhaltenen aufgeblähten Polymerteilchen einen Metallcarbonyl-, Organocarbonyl- oder Kohlenwasserstoffkomplex, der in der organischen Flüssigkeit löslich ist, ein, gemäß einem Gewichtsverhältnis von Metallkomplex/Polymer in dem Bereich von 0,5/100 bis 200/100, und man bewirkt eine Thermolysereaktion des Komplexes, wobei diese Reaktion unter Reduktionsatmosphäre durchgeführt wird, wenn der Komplex ein Metallkohlenwasserstoffkomplex ist,

c) man entfernt die organische Aufblähflüssigkeit,

d) man suspendiert gegebenenfalls die so erhaltenen zusammengesetzten kompakten Teilchen erneut in einer das Polymer nicht-aufblähenden Flüssigkeit.

6. Verfahren nach einem der Ansprüche 3 bis 5, dadurch gekennzeichnet, daß sie kalibriert sind, daß heißt, daß sie eine einheitliche Größe in dem Bereich von 0,5 bis 100 Mikron und eine Standardabweichung im Durchmesser unter 5 % aufweisen.

7. Verfahren nach einem der Ansprüche 3 bis 6, dadurch gekennzeichnet, daß sie zu 97 bis 74 Gewichts-% aus der Matrix und zu 3 bis 26 Gewichts-% aus Metall bestehen.

8. Verfahren nach einem der Ansprüche 3 bis 7, dadurch gekennzeichnet, daß die Größe der Kristallite des Metalls in dem Bereich von $3 \times 10^{-6}$ bis $3 \times 10^{-5}$ mm ist.

9. Verfahren nach einem der Ansprüche 3 bis 8, dadurch gekennzeichnet, daß die Teilchen in Form von wäßrigen Dispersionen vorliegen, welchen 1 bis 50 Gewichts-% der kalibrierten zusammengesetzten Teichlen umfassen.

10. Verfahren zur Herstellung von wäßrigen Dispersionen aus zusammengesetzten Teilchen, definiert in Anspruch 3, dadurch gekennzeichnet, daß:

a) man in einer organischen Flüssigkeit, die Lösungsmittel ist für Metallcarbonyl-, Organocarbonyl- oder Kohlenwasserstoffkomplexe, welche bei einer Temperatur niedriger als der Siedepunkt der organischen Flüssigkeit thermisch zersetzbar sind, wasserfreie und kalibrierte Polymerteilchen dispergiert, die in der genannten organischen Flüssigkeit unlöslich sind und welche nucleophile, an die genannten Komplexe koordinierbare Stellen tragen, wobei die Größe der genannten Teilchen in dem Bereich von 0,1 Mikron bis 1 mm liegt, die wobei Beschaffenheit des Polymeren, aus welchem die Teilchen bestehen, eine solche ist, daß die Partikel geeignet sind, in Gegenwart der organischen Flüssigkeit auf das 0,1- bis 50-fache ihres Volumens aufgebläht zu werden;

b) man in die Dispersion der so erhaltenen aufgeblähten Polymerteilchen einen Metallcarbonyl-, Organocarbonyl- oder Kohlenwasserstoffkomplex, der in der organischen Flüssigkeit löslich ist, einführt, gemäß einem Gewichtsverhältnis von Metallkomplex/Polymer in dem Bereich von 0,5/100 bis 200/100, und man eine Thermolysereaktion des Komplexes bewirkt, wobei diese Reaktion unter Reduktionsatmosphäre durchgeführt wird, wenn der Komplex ein Metallkohlenwasserstoffkomplex ist,

c) man die organische Aufblähflüssigkeit entfernt,

d) man die erhaltenen zusammengesetzten Teilchen mit einer nicht-aufblähenden, intermediär mit der organischen Aufblähflüssigkeit und mit Wasser mischbaren Flüssigkeit wäscht,

e) man die nicht-aufblähende Flüssigkeit entfernt,

f) man die erhaltenen zusammengesetzten Teilchen in Wasser dispergiert, durch Waschen mit Wasser bis in Wasser eine Konzentration der Teilchen in dem Bereich von 1 bis 50 Gewichts-% erhalten wird.

11. Verfahren nach einem der einem der voranstehenden Ansprüche, dadurch gekennzeichnet, daß in Schritt a) die Größe der Polymerteilchen in dem Bereich von 0,5 bis 100 Mikronen liegt.

12. Verfahren nach einem der voranstehenden Ansprüche, dadurch gekennzeichnet, daß in Schritt a) die Polymerteilchen geeignet sind, in Gegenwart der organischen Flüssigkeit auf das 3- bis 10-fache ihres Volumens aufgebläht zu werden.

13. Verfahren nach einem der voranstehenden Ansprüche, dadurch gekennzeichnet, daß in Schritt b) das Gewichtsverhältnis Metallkomplex/Polymer in dem Bereich von 3/100 bis 35/100 liegt.

14. Verfahren nach einem der voranstehenden Ansprüche, dadurch gekennzeichnet, daß das Polymer besteht aus:

- 30 bis 99 Gewichts-% mindestens eines monoethylenischen Monomeren, 0,5 bis 50 Gewichts-% mindestens eines polyethylenisch vernetzbaren Monomeren und 0,5 bis 30 Gewichts-% mindestens eines nucleophil koordinierbaren Monomeren.

15. Verfahren nach Anspruch 14, dadurch gekennzeichnet, daß das Polymer besteht aus:

- 50 bis 95 Gewichts-% mindestens eines monoethylenischen Monomeren,
- 2 bis 20 Gewichts-% mindestens eines Polyethylenisch vernetzbaren Monomeren
- 3 bis 30 Gewichts-% mindestens eines nucleophil koordinierbaren Monomeren.

EP 0 285 502 B1

16. Verfahren nach Anspruch 14 oder 15, dadurch gekennzeichnet, daß das monoethylenische Monomere Styrol, ein Styrolderivat, ein Acrylsäure- oder Methacrylsäureester oder -amid, Acryl- oder Methacrylsäure, eine monoethylenische Disäure, ein Vinylpyrrolidon, ein Vinylester, Vinyl- oder Vinylidenchlorid ist.

17. Verfahren nach einem der Ansprüche 14 bis 16, dadurch gekennzeichnet, daß das polyethylenische Monomere Divinylbenzol, ein Divinylbenzolderivat, ein konjugiertes Dien, ein Polyallylderivat, ein Polyolacrylat oder -methacrylat, Methylen-bis(acrylamid),Bis(acrylamid)essigsäure ist.

18. Verfahren nach einem der Ansprüche 14 bis 17, dadurch gekennzeichnet, daß das nucleophil koordinierbare Monomer Vinylpyridin, ein Di(ethyl)aminoalkylacrylat oder -methacrylat, ein Di(ethyl)aminoalkylacrylamid oder -methacrylamid, Allylamin, Ethylenimin, Acrylnitril oder Methacrylnitril, 1-Vinylimidazol, ein Dialkylaminomethylstyrol ist.

19. Verfahren nach einem der voranstehenden Ansprüche, dadurch gekennzeichnet, daß die organische Aufblähflüssigkeit ein aromatisches Derivat ist, gegebenenfalls chloriert, ein aliphatischer oder cycloaliphatischer Kohlenwasserstoff, ein Dialkylether, ein Alkohol, ein Ester, wobei der Siedepunkt über der Zersetzungstemperatur des Komplexes liegt.

20. Verfahren nach einem der voranstehenden Ansprüche, dadurch gekennzeichnet, daß die Konzentration der Polymerteilchen in der organischen Aufblähflüssigkeit in Schritt a) von 1 bis 35 Gewichts-% reicht.

21. Verfahren nach einem der voranstehenden Ansprüche, dadurch gekennzeichnet, daß der Carbonylkomplex Organocarbonyl vom aliphatischen, cycloaliphatischen oder aromatischen Typ, Kohlenwasserstoff vom aliphatischen, cycloaliphatischen Typ mit Metallen der Gruppen VIa, VIIa und VIII des Periodensystems der Elemente ist.

22. Verfahren nach Anspruch 21, dadurch gekennzeichnet, daß die Komplexe die Formeln haben $Cr(CO)_6$; $Mo(CO)_6$; $W(CO)_6$; $(Mn)_2(CO)_{10}$; $(Re)_2(CO)_{10}$; $Fe(CO)_5$; $(Fe)_2(CO)_9$; $(Fe)_3(CO)_{12}$; $(Co)_2(CO)_8$; $(Co)_4(CO)_{12}$; $Ni(CO)_4$; $(Rh)_6(CO)_{12}$; $(Rh)_4(CO)_{12}$; $(Ir)_4(CO)_{12}$; $(Os)_3(CO)_{12}$; $(Ru)_3(CO)_{12}$ (Methylcyclopentadienyl)-$Mn(CO)_3$; (Cyclopentadienyl)$Mn(CO)_3$; (Cyclopentadienyl)$Co(co)_2$;(Cyclopentadienyl)$Re(CO)_3$ (Bi-$\eta^5$-cyclopentadienyl)Ni oder -Fe.

23. Verfahren nach einem der voranstehenden Ansprüche, dadurch gekennzeichnet, daß die Thermolysereaktion bei einer Temperatur von 80 bis 200 °C durchgeführt wird.

24. Verfahren nach Anspruch 23, dadurch gekennzeichnet, daß die Thermolysereaktion bei einer Temperatur in der Nähe des Siedepunktes der organischen Aufblähflüssigkeit durchgeführt wird.

25. Verwendung der kompakten zusammengesetzten Polymer/Metallteilchen als solche oder in einer wäßrigen Dispersion, erhalten nach dem Verfahren nach einem der voranstehenden Ansprüche in der Biologie.

26. Verwendung der kompakten zusammengesetzten Polymer/Metallteilchen als solche oder in wäßrigen Dispersionen, erhalten nach einem der Ansprüche 3 bis 24, als feste Phase in der Biologie in diagnostischen Tests.

31